Europäisches Patentamt

European Patent Office

Office européen des brevets

(11) Publication number: 0 266 170
A2

# EUROPEAN PATENT APPLICATION

(21) Application number: 87309481.7

(22) Date of filing: 27.10.87

(51) Int. Cl.⁴: C 07 K 7/10
A 61 K 37/02, C 07 K 1/00

(30) Priority: 28.10.86 JP 257179/86

(43) Date of publication of application:
04.05.88 Bulletin 88/18

(84) Designated Contracting States:
AT BE CH DE ES FR GB GR IT LI LU NL SE

(71) Applicant: Takeda Chemical Industries, Ltd.
27, Doshomachi 2-chome Higashi-ku
Osaka-shi Osaka, 541 (JP)

(72) Inventor: Fujino, Masahiko
10-7, Hibarigaoka 2-chome
Takarazuka Hyogo 665 (JP)

Wakimasu, Mitsuhiro
B-510, 17 Yamatecho 3-chome Suita
Osaka 564 (JP)

Nishikawa, Kohei
5-19, Oharano-kamisatotorimicho
Nishikyo-ku Kyoto 610-11 (JP)

(74) Representative: Lewin, John Harvey et al
Elkington and Fife High Holborn House 52/54 High
Holborn
London WC1V 6SH (GB)

(54) Peptide derivative and its production.

(57) A peptide derivative of the formula

A-Cys-B-Arg-C-Asp-Arg-Ile-Gly-Ala-E-Cys-Asn-Ser-Phe-
Arg-Tyr-OH                              (I)

wherein A is hydrogen or a hydrocarbon acyl having 2 to 10
carbon atoms which may be substituted with an amino group, B
is Phe, Gly, Phe-Gly, Gly-Gly or Phe-Gly-Gly, C is a neutral
α-amino acid residue, and E is Gln, Gln-Ser or Gln-Ser-Gly,
respectively or a salt thereof is useful as a reagent for
elucidating the morbid physiological role of αhANP, and also
used for the therapy of hypotension, peripheral circulatory
insufficiency of mammals (e.g. mice, rats, rabbits, dogs, cats
and humans, etc.).

**Description**

Peptide Derivative and Its Production

This invention relates to an atrial natriuretic peptide derivative which is useful as a reagent for elucidating the morbid physiological role of atrial natriuretic peptides and also as medicines such as a therapeutic agent for hypotension, cardiac and cerebral circulatory diseases, etc. an anti-diuretic agent, a therapeutic agent for cardiac and cerebral circulatory diseases, etc.

Recently, three types of peptides having a strong atrial natriuretic action were isolated from human atrial tissue, and their structures were elucidated. Among these peptides, the one having the smallest molecular weight consists of 28 amino acid fragments, which was named α-human atrial natriuretic polypeptide (hereinafter abbreviated as α-hANP) [Biochemical Biophysical Research Communications, 118, 131-139 (1984)].

The structural formula of α-hANP is as shown below.

```
       1                5        ┌──────────10─────────────┐
     H-Ser-Leu-Arg-Arg-Ser-Ser-Cys-Phe-Gly-Gly-Arg-Met-Asp-

    ┌─────15──────────────20─────────────┐
    Arg-Ile-Gly-Ala-Gln-Ser-Gly-Leu-Gly-Cys-Asn-Ser-Phe-Arg-
                                                   25

    28
    Tyr-OH
```

While studies conducted later on the relationship between the structure of α-hANP and the activity thereof revealed a variety of α-hANP derivatives having a natriuretic action [PEPTIDE CHEMISTRY 1984, Edited by N.IZUMIYA, p229-234, p241-246(1984)], no compound showing a hypertensive action has been known yet.

In recent years, the chemical structure of α-hANP having hypotensive and natriuretic actions has been elucidated, and α-hANP or its derivatives are expected to be novel therapeutic drugs for hypertension, cardiac•cerebral circulatory diseases. It is, however, hardly considered that the mechanism of the action of α-hANP has been elucidated. For the elucidation, it has been strongly desired to develop a novel α-hANP antagonist.

Aiming at development of a novel α-hANP antagonist, the present inventors synthesized α-hANP derivatives whose cyclic moiety was curtailed, and found that the novel compounds of the present invention have a strong hypertensive action, thus the present invention being completed.

More specifically, the present invention relates to
(1) Peptide derivative of the formula (I):

```
    ┌──────────────────────────────────────────┐
    A-Cys-B-Arg-C-Asp-Arg-Ile-Gly-Ala-E-Cys-Asn-Ser-Phe-

    Arg-Tyr-OH                                          (I)
```

wherein A is hydrogen or a hydrocarbon acyl having 2 to 10 carbon atoms which may be substituted with an amino group, B is Phe, Gly, Phe-Gly, Gly-Gly or Phe-Gly-Gly, C is a neutral α-amino acid residue, and E is Gln, Gln-Ser or Gln-Ser-Gly, respectively or a salt thereof, and

(2) a method for producing a peptide derivative of the general formula (I) or a salt thereof, which comprises subjecting a peptide derivative of the general formula (II):
A-Cys-B-Arg-C-Asp-Arg-Ile-Gly-Ala-E-Cys-Asn-Ser-Phe-Arg-Tyr-OH    (II)
wherein A, B, C and E are of the same meaning as defined above or a salt thereof, to oxidation.

In the present specification, amino acids and peptides are sometimes shown by abbreviations which are commonly used in the relevant field or employed by the IUPAC-IUB Commission on Biochemical Nomenclature, which are exemplified as below. It should be noted that these abbreviations mean L-form unless optical configuration is specified.
Ala : Alanine
Asp : Aspartic acid
Asn : Asparagine
Cys : Cysteine
Phe : Phenylalanine
Gly : Glycine

Ile : Isoleucine
Leu : Leucine
Met : Methionine
Ser : Serine
Tyr : Tyrosine
Arg : Arginine
Val : Valine
nor-Leu : nor-Leucine
Lys : Lysine
Thr : Threonine
Gln : Glutamine
Trp : Tryptophan
His : Histidine
Pro : Proline

The protective groups and reagents mentioned frequently in this text are shown by the following abbreviations.

Z : Carbobenzoxy
Boc : t-Butoxycarbonyl
Bz$\ell$ : Benzyl
MBz$\ell$ : p-Methoxybenzyl
Pme : Pentamethylbenzenesulfonyl
OBz$\ell$ : Benzyl ester
OBu$^t$ : t-Butyl ester
DCC : N,N'-dicyclohexylcarbodiimide
DCU : N,'-dicyclohexylurea
HONB : N-hydroxy-5-norbornene-2,3-dicarboxyimide
-ONB : HONB ester
HOBt : 1-Hydoxybenzotriazole
TEA : Triethylamine
CHA : Cyclohexylamine
DCHA : Dicyclohexylamine
p-Tos-OH : Paratoluenesulfonic acid
AcOH : Acetic acid
TFA : Trifluoroacetic acid
HF : Anhydrous hydrogen fluoride
HC$\ell$ : Hydrochloric acid
AcOEt : Ethyl acetate
DMF : N,N-dimethylformamide
MeOH : Methanol
Aib : Aminoisobutyric acid
Gaba : γ-Aminobutyric acid
Ava : 5-Aminovaleric acid
Aca : 8-Aminocapric acid

In the present invention, the hydrocarbon acyl in the hydrocarbon acyl groups having 2 to 10 carbon atoms which may be substituted with an amino group, shown by A, includes aliphatic, aromatic or aliphatic-aromatic ones.

The aliphatic hydrocarbon acyl may be saturated or unsaturated straight-chained, branched or cyclic ones, as exemplified by acetyl, propionyl, butyryl, isobutyryl, valeryl, isovaleryl, pivaloyl, caproyl, caprylyl, capryl, cyclobutanecarbonyl, cyclopentanecarbonyl, cyclohexanecarbonyl, acryloyl,propioloyl, methacryloyl and crotonoyl, etc.

The aromatic hydrocarbon acyl or the aliphatic-aromatic hydrocarbon acyl is exemplified by benzoyl, phenylacetyl, phenylpropionyl, etc.

The hydrocarbon acyl may be substituted with one or two amino groups.

The amino-substituted hydrocarbon acyl is exemplified by amino acid residues such as Gly, Ala, Val, Leu, Ile, nor-Leu, Phe, Lys, Aib, 1-aminocyclobutanecarboxylic acid, 1-amino-1-cyclopentanecarboxylic acid, 1-amino-1-cyclohexanecarboxylic acid, 2-phenyl-Gly, 2-aminocaproic acid, 2-aminocapric acid, β-ala, Gaba, Ava, 6-aminocaproic acid and Aca, etc. Among them, Gly, Ala, β-Ala, Gaba, Aib, Phe, Val, Leu, nor-Leu, etc. are preferable, and when an optical isomer is present for the amino acid, it may take either L-form or D-form.

The neutral α-amino acid in the neutral α-amino acid residue shown by C in the present invention includes ones having 2 to 10 carbon atoms, which are exemplified by Gly, Ala, Val, Leu, Ile, nor-Leu, Met, Met sulfoxide, Met sulfone, Ser, Thr, Asn, Gln, Phe, Tyr, Trp, His, Pro, Cys, Aib, 1- amino-1-cyclobutanecarboxylic acid, 1-amino-1-cyclopentanecarboxylic acid, 2-cyclohexylglycine 1-amino-1-cyclohexanecarboxylic acid 2-cyclohexylglycine, 2-aminocaproic acid and 2-aminocapric acid, etc. Among them, are preferable Val, Leu, Ile, nor-Leu, Met, Phe, 2-cyclohexylglycine, 2-phenylglycine, etc.

Of the above-mentioned amino acids, those having optical isomers may take either L-form or D-form.

3

The method for producing the peptide derivatives (I) of the present invention will be described below.

The peptide derivatives (I) of the present invention can be produced by a per se conventional method for peptide synthesis. The method may be any of the solid-phase and liquid-phase type, and the latter one is, in most cases, advantageous. Such methods of peptide synthesis can be conducted in accordance with any of the conventional methods, which include, for example, those described in Peptide Synthesis, M. Bodansky and M. A. Ondetti, Interscience, New York, 1966; The Proteins, Vol.2, F. M. Finn and K. Hofmann, edited by H. Nenrath and R. L. Hill, Academic Press Inc., New York, 1976; and "The Basis and Experiment of Peptide Synthesis", Nobuo Izumiya et al.,Maruzen, 1985, as exemplified by the azide method, the chloride method, the acid anhydride method, the mixed acid anhydride method, the DCC method, the active ester method, the method using Woodward reagent K, the carbodiimidazole method, the oxidation-reduction method and the DCC/HONB method, etc.

The compound (I) of the present invention can be produced by subjecting a material having reactive carboxyl groups, the material corresponding to one of the two fragments severed at an optional position of the peptide linkage, and a material having reactive carboxyl groups, the material corresponding to the other fragment, to condensation by a conventional means in the field of peptide synthesis, followed by subjecting the condensate to oxidation, provided that, when the condensate has a protective group, the protective group is removed before-hand by a conventional means.

Protection of functional groups which are not to be involved in the reaction of the starting material, the protective groups themselves, and elimination of the protective groups, and activation of functional groups to be involved in the reaction can be suitably selected from among conventional ones.

Examples of protective groups of the amino groups of the starting materials include carbobenzoxy, t-butyloxycarbonyl, t-amyloxycarbonyl, isobornyloxycarbonyl, p-methoxybenzyloxycarbonyl, 2-chloro-benzyloxycarbonyl, adamantyloxycarbonyl, a trifluoroacetyl, phthalyl, formyl, o-nitrophenylsulfenyl, diphenylphosphinothioyl, 9-fluorenylmethyloxycarbonyl, etc.

Examples of protective groups of the carboxyl groups include alkyl ester (e.g. ester groups of methyl, ethyl, propyl, butyl, t-butyl, etc.), benzyl ester, p-nitrobenzyl ester, p-methoxybenzyl ester, p-chlorobenzyl ester, benzhydryl ester, phenacyl ester, carbobenzoxyhydrazide, t-butyloxycarbonylhydrazide, tritylhydrazide, etc.

Examples of protective groups of thiol of cysteine include para-methoxybenzyl, 4-methylbenzyl, benzyl, t-butyl, adamantyl, trityl, acetamidomethyl, carbomethoxysulfenyl, 3-nitro-2-pyridinesulfenyl, etc.

Examples of protective groups of the guanidino group of arginine include nitro, tosyl, p-methoxybenzenesulfonyl, mesitylenesulfonyl, pentamethyl, benzenesulfonyl, 4-methoxy-2,3,6-trimethylbenzenesulfonyl, carbobenzoxy, isobornyloxycarbonyl, adamantyloxycarbonyl, etc. The guanidino group may be protected in the form of a salt of an acid (e.g. benzenesulfonic acid, toluenesulfonic acid, hydrochloric acid, sulfuric acid, etc.).

The hydroxyl group of serine may be protected by, for example, esterification or etherification. Examples of groups suitable for this esterification include lower alkanoyl groups such as acetyl group; aroyl groups such as benzoyl group; groups derivable from carbonic acid such as benzyloxycarbonyl group and ethyloxycarbonyl; etc. Examples of groups suitable for the etherification include benzyl groups, tetrahydropyranyl group, t-butyl group, etc. However, protection of the hydroxyl group of serine is not always necessary.

Examples of protective groups of the phenolic hydroxyl group of tyrosine include benzyl, 2,6-dichlorobenzyl, 2-nitrobenzyl, etc., but it is not always necessary to protect the phenolic hydroxyl group.

Examples of β-carboxyl-protecting groups of aspartic acid include benzyl ester, p-nitrobenzyl ester, p-chlorobenzyl ester, alkyl ester (e.g. t-butyl ester, cyclopentyl ester, cyclohexyl ester and cycloheptyl ester), etc. The methionine may be protected in the form of sulfoxide.

Examples of the activated carboxyl group of the starting material include corresponding acid anhydrides, azides, active esters [esters with alcohol (e.g. pentachlorophenol, 2,4,5-trichlorophenol, 2,4-dinitrophenol, cyanomethyl alcohol, p-nitrophenol, N-hydroxy-5-norbornene-2,3-dicarboxyimide, N-hydroxysuccinimide, N-hydroxyphthalimide or N-hydroxybenztriazole)]. Examples of the activated amino group of the starting material include corresponding phosamides.

The condensation can be carried out in the presence of a solvent. The solvent can be suitably selected from those known as useful in the peptide condensation. Examples of such solvents include anhydrous or hydrous dimethylformamide, dimethylsulfoxide, pyridine, chloroform, dioxane, dichloromethane, tetrahydrofuran, acetonitrile, ethyl acetate, N-methylpyrrolidone or suitable mixtures of them.

Reaction temperatures can be suitably selected from the range known as employable in the reaction for forming peptide-linkage, usually the range from about -20°C to about 30°C. The precursor (protected peptide) of the compound of the present invention can also be easily produced by the solid-phase synthesis.

The protected peptide thus obtained is then subjected to deprotecting reaction. This reaciton depends on the type of protective groups then used, but, in any case, it is favorable in industrial manufacture to eliminate all the protective groups in a single step without affecting the peptide linkage. Protective groups are therefore selected taking this aspect into consideration. In the case of cysteine-containing peptide, however, it is sometimes more favorable to eliminate the protective groups in two steps, i.e., to first eliminate the protective groups other than those for thiol, then to eliminate the protective groups for thiol,in view of the easiness of purification. Examples of such thiol-protective groups as above include acetamidomethyl group, 3-nitro-2-pyridinesulfenyl group, etc.

Possible means for eliminating protective groups include acid treatment using, for example, anhydrous hydrogen fluoride, methanesulfonic acid, trifluoromethanesulfonic acid, trifluoroacetic acid or a mixed solution thereof; mention may also be made of reduction in liquid ammonia by means of sodium. The above deprotecting reaction by acid treatment is normally carried out at an appropriate temperature of -20 to 40°C; the addition of a cation scavenger such as anisole, phenol, thioanisole or dimethylsulfide is effective in the acid treatment. Protective groups for thiol that are stable to acid treatment such as acetamidomethyl and 3-nitro-2-pyridinesulfenyl can be eliminated with iodine or mercury acetate in the former case, and with mercaptoethanol or the like in the latter case.

In carrying out the above-mentioned deprotection, when the neutral α-amino acid residue shown by C is Cys, only the protective groups for amino acids other than the said Cys are previously eliminated, then the resultant is subjected to oxidation by the method described below, followed by eliminating the protective groups for the said Cys.

The thiol peptide thus obtained by eliminating the protective groups of the protected peptide is subjected to oxidation.

Suitable methods of the oxidation include those which are carried out in a solvent such as water using air, potassium ferricyanide, iodine, diiodoethane or the like. It is normally desirable that the above oxidation be carried out by the high dilution method at an appropriate temperature of about 0°C to about 40°C at a pH of about 6 to about 7.5 for about 5 minutes to about 10 hours.

The peptide derivative (I) thus produced is recovered by means of a peptide separation such as extraction, partition, reprecipitation, recrystallization, column chromatography, etc., after completion of the reaction.

The peptide derivative (I) of the present invention may also be obtained in the form of an acid addition salt, specifically a pharmacologically acceptable acid addition salt, by a per se known method. Examples of such acid addition salts include salts of inorganic acids (e.g. hydrochloric acid, sulfuric acid and phosphoric acid) or organic acids (e.g. acetic acid, propionic acid, citric acid, tartaric acid, malic acid, oxalic acid and methanesulfonic acid), etc.

The pharmacological action of the peptide derivatives of the present invention is described below.

## Hypertensive action in spontaneously hypertensive rats (SHR)

Three male SHRs at the age of 20 weeks in each group were anesthetized with pentobarbital (50 mg/kg, intraperitoneal administration); SP-10 and SP-45 polyethylene tubes (manufactured by Natsume Seisakusho, Japan) were inserted respectively into the groin artery and vein, and through each of these a cannula was passed to the neck site via the dorsal subcutaneous tissue, and one end of the cannula was fixed there. These rats were used in experiments the fol lowing day.

As peptide derivatives of the present invention, [des Leu[21],des Gly[22]]-α-hANP[7-28] obtained in Example 1 described later, [des Ser[19], des Glyz[20,22],des Leu[21]]-α-hANP[7-28] obtained in Example 3 and [Cys[9],des Leu[21],des Gly[22]]-α-hANP[8-28] obtained in Example 4, and as a control, α-hANP[7-28], as solution in physiological saline, were each intravenously administered 100μg/250μℓ/kg of body weight. Hypertensive activities are shown as a degree of the maximum increase in blood pressure (difference from the preadministration value) that occurred after the administration of the test compounds, provided that, in the case of α-hANP [7-28], the activity is shown as a degree of maximum decrease in blood pressure.

|  | Degree of Increase in Blood Pressure (mmHg) |
| --- | --- |
| α-hANP [7-28] | -22 |
| [des Leu[21],des Gly[22]]-α-hANP [7-28] | +21 |
| [des Ser[19],des Gly[20,22],des Leu[21]]-α-hANP [7-28] | +12 |
| [Cys[9],des Leu[21],des Gly[22]]-α-hANP [8-28] | +16 |

The peptide derivatives (I) of the present invention and salts thereof inhibit vasorelaxant action of α-hANP in vitro assay using thoracic aorta pieces of a rabbit, and they further exhibit hypertensive action as is apparent from the afore-mentioned pharmacological data.

In addition, the peptide derivatives (I) of the present invention and salts thereof are generally low in toxicity.

Therefore, since the peptide derivatives (I) and their salts of the present invention have hypertensive action, they are useful as reagents for elucidating the morbid physiological role of α-hANP, and also useful for the therapy of hypotension, peripheral circulatory insufficiency, etc. of mammals (e.g. mice, rats, rabbits, dogs, cats and humans, etc.).

The peptide derivatives of the present invention can be administered in the free form or as an acid addition salt thereof. The dosage is normally an appropriate level in the range of 1 ng to 10 mg per kg of body weight, calculated as a free compound, for both free compounds and acid addition salts of the derivatives (I). The derivatives of the present invention are principally parenterally administered (e.g. by intravenous or subcutaneous injection, intraventricular, intraspinal, nasal or rectal administration); in some cases, however, they are orally administered.

Dosage forms which can be used include injection and suppository. The derivatives of the present invention are stable as substances, and, therefore, they may be stored as a physiological saline solution, but they may also be lyophilized in the presence of mannitol or sorbitol and put in ampoules for extemporaneous dissolution.

The present invention will be illustrated more specifically by way of the following examples. Sephadex LH-20 employed for purification of ultimate products is manufactured by Pharmacia S.A. (Sweden). Purity of the compounds produced was determined by means of a thin-layer chromatography using Kieselguhr 60F-254 manufactured by E. Merck AG (W. Germany). Developing solvents are as follows:

Rf[1]: chloroform - methanol (19:1)
Rf[2]: chloroform - methanol - acetic acid (9:1:0.5)
Rf[3]: chloroform - methanol - water (7:3:0.5)
Rf[4]: ethyl acetate - n-butanol - acetic acid - water (1:1:1:1)

Example 1 Production of [des Leu[21],des Gly[22] ]-α-hANP[7-28]

(I) Production of Boc-Arg(Pme)-Tyr(Bzℓ)-OBzℓ

In 30 mℓ of TFA was dissolved 5.6 g of Boc-Tyr(Bzℓ)-OBzℓ. The solution was then concentrated, to which was added 10 mℓ of 3N-HCℓ/dioxane. To the mixture was added ether, then precipitating crystals were collected by filtration and dried. The crystals were dissolved in 50mℓ of DMF, and the solution was cooled with ice. To the solution were added 1.9 mℓ of TEA, 5.8 g of Boc-Arg(Pme)-OH, 1.78 g of HOBt and 2.72 g of DCC. The mixture was stirred overnight. The resultant DCU was separated by filtration, and the filtrate was concentrated. The concentrate was dissolved in AcOEt, and the solution was washed with 4% aqueous solution of NaHCO$_3$ and then 10% aqueous solution of citric acid, then with water, followed by drying over Na$_2$SO$_4$. The drying agent was filtered off, and the filtrate was concentrated. To the concentrate was added ether, and the resulting precipitates were collected by filtration. Yield (%) 9.15 g (92.0%) m.p. 114 to 116°C Rf[1]: 0.39 [α] $_D^{25}$   -5.2° (c = 1.0, in DMF)
Analysis Calc'd for C$_{45}$ H$_{57}$ N$_5$O$_8$S : C,65.27; H,6.94; N,8.46; S,3.87
Found : C,65.29; H,7.05; N,8.43; S,3.86

(II) Production of Boc-Phe-Arg(Pme)-Tyr(Bzℓ)-OBzℓ

By the same procedure as in Example 1-(I), 9.0 g of Boc-Arg(Pme)-Tyr(Bzℓ)-OBzℓ was treated with TFA. The resultant amine component was dissolved in 100 mℓ of acetonitrile. The solution was cooled with ice, to which was added 4.4 mℓ of TEA. To the mixture was added Boc-Phe-ONB (prepared from 3.2 g of Boc-Phe-OH, 2.4 g of HONB and 2.7 g of DCC). The whole mixture was stirred overnight and then there was added 1 mℓ of (CH$_3$)$_2$N(CH$_2$)$_3$NH$_2$. The mixture was shaken for 30 minutes, followed by concentration. The concentrate was dissolved in 300 mℓ of AcOEt, and the solution was washed with 4% aqueous solution of NaHCO$_3$ and then 10% aqueous solution of citric acid, followed by drying over Na$_2$SO$_4$. The resultant was concentrated. To the concentrate was added ether. The resulting precipitates were collected by filtration. Yield 10.2 g (95.9%) m.p. 136 to 137°C Rf[1]: 0.39 [α] $_D^{25}$   -8.5° (c = 0.9, in DMF)
Analysis Calc'd for C$_{54}$ H$_{66}$ N$_6$O$_9$S : C,66.51; H,6.82; N,8.62; S,3.29
Found : C,66.56; H,6.91; N,8.61; S,3.22

(III) Production of Boc-Ser(Bzℓ)-Phe-Arg(Pme)-Tyr(Bzℓ)-OBzℓ

By the same procedure as in Example 1-(I), 9.8 g of Boc-Phe-Arg(Pme)-Tyr(Bzℓ)-OBzℓ was treated with TFA. The resultant amine component was dissolved in 100 mℓ of DMF. The solution was cooled with ice, to which was added 3 mℓ of TEA. To the solution was added BOC-Ser(Bzℓ)-ONB (prepared from 3.6 g of Boc-Ser(Bzℓ)-OH, 2.4 g of HONB and 2.7 g of DCC). The whole mixture was stirred overnight and then there was added 0.5 mℓ of (CH$_3$)$_3$N(CH$_2$)$_3$NH$_2$. The mixture was shaken for 30 minutes, followed by concentration. The concentrate was dissolved in 300 mℓ of AcOEt, and the solution was washed with 4% aqueous solution of NaHCO$_3$ and then 10% aqueous solution of citric acid, followed by drying over Na$_2$SO$_4$. The resultant was concentrated. To the concentrate was added ether. The resulting precipitates were collected by filtration. Yield 10.9 g (94.4%) m.p. 117 to 119°C, Rf[1] 0.41 [α] $_D^{25}$   -7.7° (c = 0.9, in DMF)
Analysis Calc'd for C$_{64}$ H$_{77}$ N$_7$O$_{11}$ S : C,66.70; H,6.73; N,8.51; S,2.78
Found : C,66.47; H,6.75; N,8.53; S,2.84

(IV) Production of Boc-Asn-Ser(Bzℓ)-Phe-Arg(Pme)-Tyr(Bzℓ)-OBzℓ

By the same procedure as in Example 1-(I), 4.5 g of Boc-Ser(Bzℓ)-Phe-Arg(Pme)-Tyr(Bzℓ)-OBzℓ was treated with TFA. The resultant amine component was dissolved in 50 mℓ of DMF. The solution was cooled with ice, to which was added 1.5 mℓ of TEA. To the mixture was added Boc-Asn-ONB (prepared from 1.2 g of Boc-Asn-OH, 1.0 g of HONB and 1.2 g of DCC). The whole mixture was stirred overnight, followed by concentration. To the concentrate was added water. The resulting precipitates were collected by filtration and dissolved in DMF. Insolubles were filtered off, and the filtrate was concentrated. To the concentrate was added ether. The resulting precipitates were collected by filtration.

6

Yield 4.75 g (96.0%) m.p. 148 to 150°C, Rf$^1$ 0.18 [α] $^{25}_D$   -21.0° (c = 0.9, in DMF)

Analysis Calc'd for $C_{68}H_{83} N_9O_{13}$ S : C,63.93; H,6.63; N,9.87; S,2.51 Found : C,63.75; H,6.66; N,10.23; S,2.58

(V) Production of Boc-Cys(MBz$\ell$)-Asn-Ser(Bz$\ell$)-Phe-Arg(Pme)-Tyr(Bz$\ell$)-OBz$\ell$

By the same procedure as in Example 1-(I), 4.6 g of Boc-Asn-Ser(Bz$\ell$)-Phe-Arg(Pme)-Tyr(Bz$\ell$)-OBz$\ell$ was treated with TFA. The resultant amine component was dissolved in 50 m$\ell$ of DMF. The solution was cooled with ice, to which was added 0.9 m$\ell$ of TEA. To the mixture was added Boc-Cys(MBz$\ell$)-ONB (prepared from 2.0 g of Boc-Cys(MBz$\ell$)-OH•CHA, 0.90 g of HONB and 1.04 g of DCC). The whole mixture was stirred overnight, followed by concentration. To the concentrate was added ether, and the resulting precipitates were collected by filtration. Yield 5.25 g (96.9%) m.p. 177 to 183°C R$^1$: 0.24 [α] $^{25}_D$   -18.6° (c = 0.9, in DMF)

Analysis Calc'd for $C_{79} H_{96} N_{10}O_{15} S_2$•$H_2O$ : C,62.92; H,6.55 N,9.29 ; S,4.25 Found : C,62.92; H,6.47 N,9.35 ; S,4.74

(IV) Production of Boc-Ser(Bz$\ell$)-Gly-OBz$\ell$

Employing 5.0 g of Boc-Ser(Bz$\ell$)-OH, 6.84 g of H-Gly-OBz$\ell$. pTos-OH, 3.4 g of HONB, 3.9 g of DCC and 2.9 m$\ell$ of TEA, the same procedure as in Example 1-(II) was used to give the end product as an oily compound. Yield 7.4 g (quantitative) Rf$^1$ 0.76

(VII) Production of Boc-Gln-Ser(Bz$\ell$)-Gly-OBz$\ell$

Employing 7.4 g of Boc-Ser(Bz$\ell$)-Gly-OBz$\ell$ and Boc-Gln-ONB (prepared from 4.1 g of Boc-Gln-OH, 3.4 g of HONB and 3.9 g of DCC), the same procedure as in Example 1-(II) was used to give the end product, which was crystallized from ether. The crystals were collected by filtration. Yield 8.8 g (91.1%) m.p. 146 to 148°C Rf$^1$ 0.13 [α] $^{25}_D$   -2.5° (c = 1.0, in DMF)

Analysis Calc'd for $C_{29} H_{38} N_4O_4$ : C,61.04; H,6.71; N, 9.82 Found : C,61.28; H,6.85; N,10.03

(VIII) Production of Boc-Ala-Gln-Ser(Bz$\ell$)-Gly-OBz$\ell$

Employing 3.7 g of Boc-Gln-Ser(Bz$\ell$)-Gly-OBz$\ell$ and Boc-Ala-ONB (prepared from 1.5 g of Boc-Ala-OH, 1.57 g of HONB and 1.80 g of DCC), the same procedure as in Example 1-(II) was used to give the end product. Yield 3.8 g (91.4%) m.p. 177 to 179°C, Rf$^2$ 0.56 [α] $^{25}_D$   -5.7° (c = 1.0 in DMF)

Analysis Calc'd for $C_{32} H_{43} N_5O_9$: C,59.89; H,6.75; N,10.91 Found : C,59.62; H,6.76; N,10.92

(IX) Production of Boc-Ala-Gln-Ser-Gly-OH

In 50 m$\ell$ of methanol was dissolved 1.5 g of Boc-Ala-Gln-Ser(Bz$\ell$)-Gly-OBz$\ell$. The solution was subjected to catalytic reduction using Pd black as the catalyst. Yield 1.1 g (quantitative) m.p. 144 to 145°C (decomp.) Rf$^3$ 0.09 [α] $^{25}_D$   -7.3° (c = 1.0, in DMF)

Analysis Calc'd for $C_{18} H_{31} N_5O_9$: C,46.85; H,6.77; N,15.18 Found : C,46.53; H,6.75; N,14.92

(X) Production of Z-Ile-Gly-OBz$\ell$

To 8.0 g of H-Gly-OBut•HC$\ell$ was added 200 m$\ell$ of acetonitrile, and the mixture was cooled with ice. To the resultant was added 6.7 m$\ell$ of TEA, to which was added Z-Ile-ONB (prepared from 14.6 g of Z-Ile-OH, 11.0 g of HONB and 12.5 g of DCC). The mixture was stirred overnight, to which was added 2.5 m$\ell$ of $(CH_3)_2N(CH_2)_3NH_2$. The whole mixture was shaken for 30 minutes, followed by concentration. The concentrate was dissolved in 300 m$\ell$ of AcOEt. The solution was washed with 4% aqueous solution of $NaHCO_3$ and then 10% aqueous solution of citric acid, which was dried over $Na_2SO_4$, followed by concentration. To the concentrate was added etherpetroleum benzin, and the resulting crystals were collected by filtration. Yield 17.8 g (85.0%) m.p. 127 to 128°C Rf$^2$ 0.81 [α] $^{25}_D$   -4.5° (c = 1.1, in DMF)

Analysis calc'd for $C_{20} H_{30} N_2O_5$ : C,63.47; H,7.99; N,7.40 Found : C,63.56; H,8.16; N,7.47

(XI) Production of Boc-Arg(Pme)-Ile-Gly-OBu$^t$

In 150 m$\ell$ of MeOH, 5.0 g of Z-Ile-Gly-OBu$^t$ was subjected to catalytic reduction using Pd black as the catalyst. The resultant amine component was dissolved in 50 m$\ell$ of DMF. To the solution were added 5.8 g of Boc-Arg(Pme)-OH and 1.8 g of HOBt. The mixture was cooled with ice, to which was added 2.8 g of DCC, followed by stirring overnight. DCU then produced was filtered off, and the filtrate was concentrated. The concentrate was dissolved in 200 m$\ell$ of AcOEt, and the solution was washed with 4% aqueous solution of $NaHCO_3$ and then 10% aqueous solution of citric acid, followed by drying over $Na_2SO_4$. The resultant was concentrated. To the concentrate was added ether, then resulting crystals were collected by filtration. Yield 8.52 g (quantitative) m.p. 115 to 118°C Rf$^1$ 0.32 [α] $^{25}_D$   -10.4° (c = 1.0, in DMF)

Analysis Calc'd for $C_{34} H_{58} N_6O_8S$ : C,57.44; H,8.22; N,11.82; S,4.51 Found : C,58.24; H,8.20; N,11.68; S,4.54

(XII) Production of Boc-Asp(OBzℓ)-Arg(Pme)-Ile-Gly-OH•CHA

In 60 mℓ of TFA was dissolved 8.50 g of Boc-Arg(Pme)-Ile-Gly-OBuᵗ. The solution was shaken for one hour at room temperature and was then concentrated. To the concentrate was added ether, then resulting precipitates were collected by filtration. The precipitates were dissolved in 100 mℓ of DMF. The solution was cooled with ice, to which was added 3.8 mℓ of TEA. To the mixture was further added Boc-Asp(OBzℓ)-ONB (prepared from 4.85 g of Boc-Asp(OBzℓ)-OH, 2.97 g of HONB and 3.40 g of DCC), followed by stirring overnight. The resultant was concentrated and acidified with citric acid and was subjected to extraction with 200 mℓ of AcOEt. The extract was washed with water, followed by drying over $Na_2SO_4$. The resultant was concentrated and there was added ether. The precipitates then formed were collected by filtration and then dissolved in AcOEt. To the solution was added 1.1 mℓ of CHA. To the mixture was further added ether, and the resulting crystals were collected by filtration, followed by recrystallization from acetonitrile. Yield 7.40 g (64.3%) m.p. 146 to 148°C Rf² 0.43 [α] $_D^{25}$ -4.2° (c=1.1, in DMF)

Analysis Calc'd for $C_{47} H_{74} N_8 O_{11} S$ : C,58,85; H,7.78; N,11.68; S,3.34 Found : C,58.59; H,7.74; N,11.61; S,3.50

(XIII) Production of Boc-Met-Asp(OBzℓ)-Arg(Pme)-Ile-Gly-OH

In 100 mℓ of AcOEt was suspended 3.0 g of Boc-Asp(OBzℓ)-Arg(Pme)-Ile-Gly-OH•CHA. To the suspension were added 4 mℓ of $1N-H_2SO_4$ and 50 mℓ of water, and the mixture was shaken, followed by washing with water and drying over $Na_2SO_4$. The resultant was concentrated and there was added 30 mℓ of TFA. The resulting amine component was dissolved in 40 mℓ of DMF. The solution was cooled with ice, to which was added 0.9 mℓ of TEA. To the mixture was added Boc-Met-ONB (prepared from 4.3 g of Boc-Met-OH•DCHA, 2.0 g of HONB and 2.3 g of DCC), followed by stirring overnight. To the resultant was added 1 mℓ of AcOH, and the mixture was concentrated. To the concentrate was added AcOEt, and the resulting crystals were collected by filtration. Yield 3.1 g (quantitative) m.p. 175 to 178°C, Rf² 0.41 [α] $_D^{25}$ -11.2° (c=1.0, in DMF)

Analysis Calc'd for $C_{46} H_{70} N_8 O_{12} S_2$ : C,55.74; H,7.12; N,11.30; S,6.47 Found : C,55.95; H,7.42; N,ll.26; S,5.96

(XIV) Production of Boc-Arg(Pme)-Met-Asp(OBzℓ)-Arg(Pme)-Ile-Gly-OH

With 20 mℓ of TFA was processed 2.6 g of Boc-Met-Asp(OBzℓ)-Arg(Pme)-Ile-Gly-OH. The resultant amine component was dissolved in 50 mℓ of DMF. The solution was cooled with ice, to which was added 1 mℓ of TEA. To the mixture was added Boc-Arg(Pme)-ONB (prepared from 1.52 g of Boc-Arg(Pme)-OH, 1.62 g of HONB and 0.71 g of DCC), followed by stirring overnight. To the resultant was added 2 mℓ of AcOH, and the mixture was concentrated. To the concentrate was added AcOEt, then the resulting precipitates were collected by filtration. Yield 3.03 g (85.2%) m.p. 230°C (decomp.) Rf² 0.37 [α] $_D^{25}$ -10.2° (c=0.2, in DMF)

Analysis Calc'd for $C_{63} H_{96} N_{12} O_{15} S_3$ : C,55.73; H,7.13; N,12.38; S,7.09 Found : C,56.33; H,7.34; N,12.17; S,6.87

(XV) Production of Boc-Gly-Gly-OBzℓ

To 22.2 g of H-Gly-OBzℓ•p-Tos-OH was added 200 mℓ of acetonitrile. The mixture was cooled with ice, to which was added 9.2 mℓ of TEA. To the whole mixture was added Boc-Gly-ONB (prepared from 10.5 g of Boc-Gly-OH, 11.9 g of HONB and 13.6 g of DCC), followed by stirring overnight. To the resultant was added 1 mℓ of $(CH_3)_2N(CH_2)_3NH_2$, and the mixture was concentrated. The concentrate was dissolved in 300 mℓ of AcOEt, and the solution was washed with 4% aqueous solution of $NaHCO_3$ and then 10% aqueous solution of citric acid, followed by drying over $Na_2SO_4$. The resultant was concentrated, to which was added petroleum benzin. Crystals then formed were collected by filtration, followed by washing with ether. Yield 18.2 g (94.1%) m.p. 81 to 82°C Rf¹ 0.53

Analysis Calc'd for $C_{16} H_{22} N_2 O_5$ : C,59.62; H,6.88; N,8.69 Found : C,59.85; H,6.96; N,8.66

(XVI) Production of Boc-Phe-Gly-Gly-OBzℓ

With 150 mℓ of TFA was processed 17.0 g of Boc-Gly-Gly-OBzℓ. The resultant amine component was dissolved in 150 mℓ of DMF. The solution was cooled with ice and neutralized with TEA, to which was added Boc-Phe-ONB (prepared from 10.0 g of Boc-Phe-OH, 7.5 g of HONB and 8.6 g of DCC). The mixture was stirred overnight, followed by concentration. The concentrate was dissolved in 300 mℓ of AcOEt, and the solution was washed with 4% aqueous solution of $NaHCO_3$ and then 10% aqueous solution of citric acid, followed by drying over $Na_2SO_4$. The resultant was concentrated, to which was added ether. Crystals then formed were collected by filtration.

Yield 16.9 g (95.5%) m.p. 94 to 96°C Rf¹ 0.41 [α] $_D^{25}$ -6.9° (c=1.0, in DMF)

Analysis Calc'd for $C_{25} H_{31} N_3 O_6$ : C,63.95; H,6.65; N,8.95 Found : C,63.96; H,6.68; N,9.20

(XVII) Production of Boc-Phe-Gly-Gly-OH•CHA

In 200 mℓ of MeOH, 7.0 g of Boc-Phe-Gly-Gly-OBzℓ was subjected to catalytic reduction using Pd black as the catalyst, followed by concentration. To the concentrate was added 1.72 mℓ of CHA, followed by crystallization from AcOEt. The crystals were collected by filtration. Yield 6.90 g (96.1%) m.p. 159 to 161°C Rf²

0.25 [α] $_D^{25}$ -10.1° (c = 1.0, in DMF)

Analysis Calc'd for $C_{24} H_{38} N_4 O_6$ : C,60.28; H,8.00; N,11.71 Found : C,59.94; H,7.99; N,11.97

(XVIII) Production of Boc-Cys(MBzℓ)-Phe-Gly-Gly-OH

Boc-Phe-Gly-Gly-OH (prepared from 6.9 g of Boc-Phe-Gly-Gly-OH•CHA and 18 mℓ of 1N-H₂SO₄) was processed with TFA. The amine component then obtained was dissolved in DMF. The solution was cooled with ice, to which was added 4.62 mℓ of TEA. To the mixture was added Boc-Cys(MBzℓ)-ONB [prepared from 6.35 g of Boc-Cys(MBzℓ)-OH•CHA, 2.88 g of HONB and 3.30 g of DCC], followed by stirring for 3 hours. To the resultant was added 0.57 mℓ of (CH₃)₂N(CH₂)₃NH₂, and the mixture was concentrated. The concentrate was dissolved in 200 mℓ of AcOEt, and the solution was washed with 4% aqueous solution of NaHCO₃ and then 10% aqueous solution of citric acid, followed by drying over Na₂SO₄. The resultant was concentrated, to which was added ether. Crystals then formed were collected by filtration. Yield 6.0 g (69.1%) m.p. 120 to 122°C Rf² 0.51 [α] $_D^{25}$ -19.8° (c = 0.9 in DMF)

Analysis Calc'd for $C_{29} H_{38} N_4 O_8 S$ : C,57.79; H,6.35; N, 9.30; S,5.32 Found : C,57.48; H,6.63; N, 9.31; S,4.89

(XIX) Production of Boc-Ala-Gln-Ser-Gly-Cys(MBzℓ)-Asn-Ser(Bzℓ)-Phe-Arg(Pme)-Tyr(Bzℓ)-OBzℓ

To 500 mg of Boc-Cys(MBzℓ)-Asn-Ser(Bzℓ)-Phe-Arg(Pme)-Tyr(Bzℓ)-OBzℓ was added 5 mℓ of TFA to make a solution. The solution was concentrated, to which was added ether. Precipitates then deposited were collected by filtration, dissolved in 3 mℓ of DMF and cooled with ice. To the resultant was added 0.47 mℓ of TEA, and the mixture was stirred, followed by addition of ether. Precipitates then deposited were collected by filtration, which were dissolved in 5 mℓ of DMF. To the solution were added 163 mg of Boc-Ala-Gln Ser-Gly-OH and 121 mg of HONB. The mixture was cooled with ice, to which was added 138 mg of DCC. The mixture was stirred for 3 days. DCU thus produced was filtered off, and the filtrate was concentrated. To the concentrate was added acetonitrile, and the resulting precipitates were collected by filtration and washed with hydrous acetonitrile. Yield 610 mg (quantitative) m.p. 210 to 221°C (decomp.) Rf² 0.29 [α] $_D^{25}$ -16.1°C (c = 1.0, in DMF)

Analysis Calc'd for $C_{92} H_{117} N_{15} O_{21} S_2$ : C,60.28; H,6.43; N,11.46; S,3.50 Found : C,60.55; H,6.83; N,11.46; S,3.80

(XX) Production of
Boc-Arg(Pme)-Met-Asp(OBzℓ)-Arg(Pme)-Ile-Gly-Ala-Gln-Ser-Gly-Cys(MBzℓ)-Asn-Ser(Bzℓ)-Phe-Arg(Pme)-Tyr(Bzℓ)-OBzℓ

Using 500 mg of the compound obtained in Example 1-(XIX), 480 mg of Boc-Arg(Pme)-Met-Asp(OBzℓ)-Arg(Pme)-Ile-Gly-OH, 98 mg of HONB and 337 mg of DCC, the desired compound was obtained by the same procedure as in Example 1-(XIX). Yield 710 mg (84.9%) m.p. 217 to 222°C (decomp.) Rf² 0.38 [α] $_D^{25}$ -15.0° (c = 0.8, in DMF)

Analysis Calc'd for $C_{150} H_{203} N_{27} O_{33} S_5$ : C,58.63; H,6.66; N,12.31; S,5.22 Found : C 58.49; H,6.83; N,11.91; S,5.15

(XXI) Production of
Boc-Cys(MBzℓ)-Phe-Gly-Gly-Arg(Pme)-Met-Asp(OBzℓ)-Arg(Pme)-Ile-Gly-Ala-Gln-Ser-Gly-Cys(MBzℓ)-Asn-Ser(Bzℓ)-Phe-Arg(Pme)-Tyr(Bzℓ)-OBzℓ

Using 600 mg of the compound obtained in Example 1-(XX), 210 mg of Boc-Cys(MBzℓ)-Phe-Gly-Gly-OH, 80 mg of HONB and 330 mg of DCC, the titled compound was obtained by the same procedure as in Example 1-(XIX). Yield 600 mg (83.8%) m.p. 217 to 225°C (decomp.)

Analysis Calc'd for $C_{174} H_{231} N_{31} O_{38} S_6$ : C,58.75; H,6.55; N,12.21; S,5.41 Found : C,59.10, H,6.86; N,12.00; S,5.21

(XXII) Production of H-Cys-Phe-Gly-Gly-Arg-Met-Asp-Arg-Ile-Gly-Ala-Gln-Ser-Cys-Asn-Ser-Phe-Arg-Tyr-OH ([des Leu²¹, des Gly²²]-α-hANP[7-28])

To 200 mg of the compound obtained in Example 1-(XXI) were added 0.3 mℓ of anisole, 0.3 mℓ of thioanisole and 3 mℓ of HF. The mixture was stirred at 0°C for 60 minutes, followed by concentration. To the concentrate was added ether to cause precipitation. The ether layer was removed by decantation. The residue was dissolved in 10 mℓ of water, followed by subjecting to ion-exchange by allowing the solution to pass through Amberlite IRA-400 (acetic acid type) (1 × 10 cm). To the resultant was added water to dilute to a volume of 1ℓ. Thus diluted aqueous solution was brought to pH 6.9 with C⁻NH₃ water, followed by addition of 500 mg of AcONH₄. The mixture was subjected to oxidation with air for 4 hours at room temperature, followed by lyophilization. Thus lyophilized material was subjected to a column (0.6 × 120 cm) of Sephadex LH-20. Elution

was conducted with 1N-AcOH, and the fractions containing the end product were combined and lyophilized. Yield 48 mg (36%)

Amino acid analysis : Asp 2.04, Ser 1.89, Glu 1.06, Gly 4.06, Ala 1.01, Half Cys 1.00, Met 1.00, Ile 1.00, Tyr 0.91, Phe 1.91, Arg 3.01

Example 2 Production of [des Gly20,22 ,Leu21 ]-α-hANP[7-28]

(I) Production of Boc-Ser(Bzℓ)-Cys(MBzℓ)-Asn-Ser(Bzℓ)-Phe-Arg(Pme)-Tyr(Bzℓ)-OBzℓ

By the same manner as in Example 1-(I), 500 mg of Boc-Cys(MBzℓ)-Asn-Ser(Bzℓ)-Phe-Arg(Pme)-Tyr(Bzℓ)-OBzℓ was processed with TFA. The resulting amine component was dissolved in 5 mℓ of DMF, and the solution was cooled with ice, to which was added 0.15 mℓ of TEA. To the mixture was added Boc-Ser(Bzℓ)-ONBℓ[prepared from 121 mg of Boc-Ser(Bzℓ)-OH, 81 mg of HONB and 93 mg of DCC], and the mixture was stirred overnight, followed by concentration. To the concentrate was added hydrous acetonitrile, then the resulting precipitates were collected by filtration. Yield 505 mg (90.4%) m.p. 180 to 184°C, $Rf^2$ 0.78 $[\alpha]_D^{25}$ -15.2° (c=0.9, in DMF)

Analysis Calc'd for $C_{89} H_{107} N_{11} O_{17} S_2$: C,64.13; H,6.47; N, 9.24; S,3.85
Found : C,63.95; H,6.59; N, 9.44; S,3.65

(II) Production of Boc-Gln-Ser(Bzℓ)-Cys(MBzℓ)-Asn-Ser(Bzℓ)-Phe-Arg(Pme)-Tyr(Bzℓ)-OBzℓ

Using 450 mg of the compound obtained in Example 2-(I) and Boc-Gln-ONB (prepared from 87 mg of Boc-Gln-OH, 70 mg of HONB and 81 mg of DCC), the same procedure as in Example 2-(I) was conducted to give the end product. Yield 480 mg (98.9%) m.p. 204 to 211°C (decomp.) $Rf^2$ 0.60 $[\alpha]_D^{25}$ -15.9° (c=1.2, in DMF)

Analysis Calc'd for $C_{94} H_{115} N_{13} O_{19} S_2$: C,62.89; H,6.46; N,10.14; S,3.57
Found : C,63.04; H,6.78; N,10.21; S,3.32

(III) Production of Boc-Ala-Gln-Ser(Bzℓ)-Cys(MBzℓ)-Asn-Ser (Bzℓ)-Phe-Arg(Pme)-Tyr(Bzℓ)-OBzℓ

Using 400 mg of the compound obtained in Example 2-(II) and Boc-Ala-ONB (prepared from 55 mg of Boc-Ala-OH, 60 mg of HONB and 68 mg of DCC), the same procedure as in Example 2-(I) was conducted to give the end product. Yield 364 mg (87.4%) m.p. 215 to 218°C (decomp.), $Rf^2$ 0.60 $[\alpha]_D^{25}$ -19.2° (c=0.9, in DMF)

Analysis Calc'd for $C_{97} H_{120} N_{14} O_{20} S_2$: C,62.43; H,6.48; N,10.51; S,3.44
Found : C,62.44; H,6.61; N,10.47; S,3.38

(IV) Production of
Boc-Arg(Pme)-Met-Asp(OBzℓ)-Arg(Pme)-Ile-Gly-Ala-Gln-Ser(Bzℓ)-Cys(MBzℓ)-Asn-Ser(Bzℓ)-Phe-Arg(Pme)-Tyr(Bzℓ)-OBzℓ

Using 300 mg of the compound obtained in Example 2-(III), 240 mg of Boc-Arg(Pme)-Met-Asp(OBzℓ)-Arg(Pme)-Ile-Gly-OH, 60 mg of HONB and 206 mg of DCC, the same procedure as in Example 1-(XD) was conducted to give the end product. Yield 415 mg (83.0%) m.p. 212 to 215°C (decomp.) $Rf^2$ 0.58 $[\alpha]_D^{25}$ -15.9° (c=1.0, in DMF)

Analysis Calc'd for $C_{155} H_{206} N_{26} O_{32} S_5$: C,59.94; H,6.69; N,11.73; S,5.16
Found : C,59.64; H,6.78; N,11.68; S,4.91

(V) Production of
Boc-Cys(MBzℓ)-Phe-Gly-Gly-Arg(Pme)-Met-Asp(OBzℓ)-Arg(Pme)-Ile-Gly-Ala-Gln-Ser(Bzℓ)-Cys(MBzℓ)-Asn-Ser(Bzℓ)-Phe-Arg(Pme)-Tyr(Bzℓ)-OBzℓ

Using 300 mg of the compound obtained in Example 2-(IV), 88 mg of Boc-Cys(MBzℓ)-Phe-Gly-OH, 36 mg of HONB and 123 mg of DCC, the same procedure as in Example 1-(XIX) was conducted to give the end product. Yield 255 mg (73.5%) m.p. 235 to 238°C (decomp.)

Analysis Calc'd for $C_{179} H_{234} N_{30} O_{37} S_6$: C,59.88; H,6.57; N,11.70; S,5.36
Found : C,60.11; H,6.85; N,11.65; S,5.18

(VI) Production of H-Cys-Phe-Gly-Gly-Arg-Met-Asp-Arg-Ile-Gly-Ala-Gln-Ser-Cys-Asn-Ser-Phe-Arg-Tyr-OH ([des Gly20,22 , des Leu21 ]-α-hANP[7-28])

By the same procedure as in Example 1-(XXII), 200 mg of the compound obtained in Example 2-(V) was treated with HF, following by subjecting the resultant to oxidation with air and purification obtain the end product. Yield 30 mg (24%)

Amino acid analysis : Asp 2.01, Ser 1.85, Glu 1.04, Gly 3.07, Ala 1.01, Half Cys 1.85, Met 0.99, Ile 1.00, Tyr 0.92, Phe 1.90, Arg 3.02

Example 3 Production of [des Ser$^{19}$ ,des Gly$^{20,22}$ ,des Leu$^{21}$]-α-hANP[7-28]

(I) Production of Boc-Gln-Cys(MBz$\ell$)-Asn-Ser(Bz$\ell$)-Phe-Arg (Pme)-Tyr(Bz$\ell$)-OBz$\ell$

Using 500 mg of Boc-Cys(MBz$\ell$)-Asn-Ser(Bz$\ell$)-Phe-Arg(Pme)-Tyr(Bz$\ell$)-OBz$\ell$ and Boc-Gln-ONB (prepared from 108 mg of Boc-Gln-OH, 88 mg of HONB and 100 mg of DCC), the same procedure as in Example 2-(I) was conducted to give the end product. Yield 520 mg (95.9%) m.p. 222 to 224°C (decomp.) Rf$^2$ 0.57 [α] $_b^{25}$ -17.6° (c = 0.6, in DMF)

Analysis Calc'd for C$_{84}$ H$_{104}$ N$_{12}$ O$_{17}$ S$_2$: C,62.36; H,6.48; N,10.39; S,3.96
Found : C,62.35; H,6.54; N,10.51; S,3.96

(II) Production of Boc-Ala-Gln-Cys(MBz$\ell$)-Asn-Ser(Bz$\ell$)-Phe-Arg(Pme)-Tyr(Bz$\ell$)-OBz$\ell$

Using 486 mg of the compound obtained in Example 3-(I) and Boc-Ala-ONB (prepared from 74 mg of Boc-Ala-OH, 77 mg of HONB and 89 mg of DCC), the same procedure as in Example 2-(I) was conducted to give the end prodcut. Yield 500 mg (98.6%) m.p. 235 to 240°C (decomp.) Rf$^2$ 0.43 [α] $_b^{25}$ -20.5° (c = 1.0, in DMF)

Analysis Calc'd for C$_{87}$ H$_{109}$N$_{13}$ O$_{18}$ S$_2$: C,61.87; H,6.50; N,10.78; S,3.80
Found : C,61.68; H,6.81;
N,10.95; S,3.55

(III) Production of
Boc-Arg(Pme)-Met-Asp(OBz$\ell$)-Arg(Pme)-Ile-Gly-Ala-Gln-Cys(MBz$\ell$)-Asn-Ser(Bz$\ell$)-Phe-Arg(Pme)-Tyr (Bz$\ell$)-OBz$\ell$

Using 300 mg of the compound obtained in Example 3-(II), 265 mg of Boc-Arg(Pme)-Met-Asp(OBz$\ell$)-Arg(Pme)-Ile-Gly-OH, 65 mg of HONB and 220 mg of DCC, the same procedure as in Example 1-(XD) was conducted to give the end prodcut. Yield 444 mg (85.7%) m.p. 215 to 235°C (decomp.) Rf$^2$ 0.48 [α] $_b^{25}$ -17.8° (c = 0.9, in DMF)

Analysis Calc'd for C$_{145}$H$_{195}$N$_{25}$ O$_{30}$ S$_5$: C,59.47; H,6.71; N,11.96; S,5.47
Found : C,59.23; H,6.82; N,11.86; S,5.01

(IV) Production of
Boc-Cys(MBz$\ell$)-Phe-Gly-Gly-Arg(Pme)-Met-Asp(OBz$\ell$)-Arg(Pme)-Ile-Gly-Ala-Gln-Cys(MBz$\ell$)-Asn-Ser (Bz$\ell$)-Phe-Arg(Pme)-Tyr(Bz$\ell$)-OBz$\ell$

Using 300 mg of the compound obtained in Example 3-(III), 90 mg of Boc-Cys(MBz$\ell$)-Phe-Gly-Gly-OH, 36 mg of HONB and 123 mg of DCC, the same procedure as in Example 1-(XIX) was conducted to give the end product. Yield 295 mg (84.7%) m.p. 222 to 226°C (decomp.)

Analysis Calc'd for C$_{169}$H$_{223}$N$_{29}$ O$_{35}$ S$_6$: C,59.47; H,6.59; N,11.90; S, 5.04
Found : C,59.78; H,6.83; N,11.90; S,5.44

(V) Production of H-Cys-Phe-Gly-Gly-Arg-Met-Asp-Arg-Ile-Gly-Ala-Gln-Ser-Cys-Asn-Ser-Phe-Arg-Tyr-OH ([des Ser$^{19}$, des Gly$^{20,22}$ ,des Leu$^{21}$ ]-α-hANP[7-28])

Using 200 mg of the compound obtained in Example 3-(IV), HF treatment, oxidation with air and purification were conducted in the same manner of Example 1-(XXII) to give the end prodcut. Yield 31 mg (24%) Amino acid analysis : Asp 2.03, Ser 0.92, Glu 1.03, Gly 3.05, Ala 1.00, Half Cys 1.87, Met 0.97, Ile 1.00, Tyr 0.93, Phe 1.93, Arg 3.03

Example 4 Production of [Cys$^9$,des Leu$^{21}$ ,des Gly$^{22}$ ]-α-hANP [8-28]

(I) Production of Boc-Cys(MBz$\ell$)-Gly-OH•DCHA

In 10 m$\ell$ of water were dissolved 1.0 g of Gly and 0.84 g of NaHCO$_3$, and the solution was cooled with ice. To the solution was added 10 m$\ell$ of acetonitrile. To the mixture was added, while stirring vigorously, Boc-Cys(MBz$\ell$)-ONB [prepared from 3.42 g of Boc-Cys(MBz$\ell$)-OH, 1.97 g of HONB and 2.27 g of DCC]. The mixture was stirred overnight, followed by concentration. To the concentrate was added 10% aqueous solution of citric acid to render the pH to acid and it was subjected to extraction with AcOEt. The extract was washed with water and dried over Na$_2$SO$_4$. The resultant was concentrated and dissolved in ether. To the ether solution was added 2 m$\ell$ of DCHA, and the resulting crystals were collected by filtration, followed by recrystallization from methanol-ether. Yield 1.77 g (33.3%) m.p. 125-130°C Rf$^2$ 0.58 [α] $_b^{25}$ -25.9° (c = 0.9, in DMF)

Analysis Calc'd for $C_{30}$ $H_{49}$ $N_3O_6S$ : C,62.15; H,8.52; N,7.25
Found : C,62.15; H,8.51; N,7.35

(II) Production of Boc-Phe-Cys(MBzℓ)-Gly-OH

With 10 mℓ of TFA was processed Boc-Cys(MBzℓ)-Gly-OH (prepared from 1.5 g of the corresponding DCHA salt). The resultant amine component was dissolved in 15 mℓ of DMF, and the solution was cooled with ice, to which was added 2 mℓ of TEA. To the mixture was added 1.2 g of Boc-Phe-ONB, which was stirred overnight, followed by addition of 0.1 mℓ of $(CH_3)_2N(CH_2)_3NH_2$. The whole mixture was concentrated. To the concentrate was added 10% aqueous solution of citric acid, followed by extraction with 50 mℓ of AcOEt. The extract was washed with water and dried over $Na_2SO_4$. The resultant was concentrated and there was added ether. Then precipitating crystals were collected by filtration. Yield 0.95 g (61.7%) m.p.112 to 115°C, Rf² 0.63 [α] $_D^{25}$ -26.2° (c=1.0, in DMF)

Analysis Calc'd for $C_{27}$ $H_{35}$ $N_3O_7S$ : C,59.43; H,6.47; N, 7.70; S,5.88
Found : C,59.56; H,6.68; N, 7.80; S,5.71

(III) Production of Boc-Phe-Cys(MBzℓ)-Gly-Arg(Pme)-Met-Asp
(OBzℓ)-Arg(Pme)-Ile-Gly-Ala-Gln-Ser-Gly-Cys(MBzℓ)-Asn-Ser(Bzℓ)-Phe-Arg(Pme)-Tyr(Bzℓ)-OBzℓ

Using 300 mg of the compound obtained in Example 1-(XX), 69 mg of Boc-Phe-Cys(MBzℓ)-Gly-OH, 50 mg of HONB and 167 mg DCC, the same procedure as in Example 1-(XIX) was conducted to obtain the end product. Yield 293 mg (87.5%) m.p. 195 to 213°C (decomp.)

Analysis Calc'd for $C_{172}H_{225}N_{30}$ $O_{37}$ $S_6$: C,59.01; H,6.57; N,12.01; S,5.50
Found : C,59.39; H,6.75; N,12.14; S,5.47

(IV) Production of H-Phe-Cys-Gly-Arg-Met-Asp-Arg-Ile-Gly-Ala-Gln-Ser-Gly-Cys-Asn-Ser-Phe-Arg-Tyr-OH ([Cys⁹,des Leu²¹ ,des Gly²² ]-α-hANP[8-28])

Using 200 mg of the compound obtained in Example 4-(III), HF treatment, air-oxidation and purification were conducted in the same manner of Example 1-(XXII) to obtain the end product. Yield 22 mg (18%)
Amino acid analysis : Asp 2.02, Ser 1.87, Glu 1.05, Gly 3.06, Ala 1.01, Half Cys 1.92, Met 0.99, Ile 1.00, Tyr 0.91, Phe 1.95, Arg 3.03

Example 5 Production of [des Glys⁹,²² ,des Leu²¹ ]-α-hANP[7-28]

(I) Production of Boc-Phe-Gly-OBzℓ

Using 6.7 g of H-Gly-OBzℓ•p-Tos-OH, 5.8 g of Boc-Phe-OH, 4.3 g of HONB and 5.0 g of DCC, the same procedure as in Example 1-(XV) was conducted to obtain the end product. Yield 6.3 g (76.4%) m.p. 132 to 134°C Rf² 0.74 [α] $_D^{25}$ -8.7° (c=1.1, in DMF)

Analysis Calc'd for $C_{23}H_{28}$ $N_2O_5$ : C,66.97; H,6.84; N, 6.79;
Found : C,66.78: H,6.85: N, 6.91

(II) Production of Boc-Phe-Gly-OH

In MeOH, 3.0 g of Boc-Phe-Gly-OBzℓ was subjected to catalytic reduction using Pd black as the catalyst to obtain the end product. Yield 2.31 g (98.6%) m.p. 158 to 160°C Rf² 0.54 [α] $_D^{25}$ -9.8° (c=1.2, in DMF)

Analysis Calc'd for $C_{16}$ $H_{22}$ $N_2O_5$ : C,59.62; H,6.88; N,8.69
Found : C,59.70; H,6.90; N,8.71

(III) Prodcution of Boc-Cys(MBzℓ)-Phe-Gly-OH

Using 1.00 g of Boc-Phe-Gly-OH, 1.16 g of Boc-Cys(MBzℓ)-OH, 0.61 g of HONB and 0.70 g of DCC, the same procedure as in Example 1-(XVIII) was conducted to obtain the end product. Yield 1.43 g (83.9%) m.p. 146 to 148°C Rf² 0.63 [α] $_D^{25}$ -29.0° (c=1.0, in DMF)

Analysis Calc'd for $C_{27}$ $H_{35}$ $N_3O_7S$ : C,59.43; H,6.47; N, 7.70; S,5.88
Found : C,60.08; H,6.74; N, 7.90; S,5.41

(IV) Production of Boc-Cys(MBzℓ)-Phe-Gly-Arg(Pme)-Met-Asp
(OBzℓ)-Arg(Pme)-Ile-Gly-Ala-Glu-Ser-Gly-Cys(MBzℓ)-Asn-Ser(Bzℓ)-Phe-Arg(Pme)-Tyr(Bzℓ)-OBzℓ

Using 300 mg of the compound obtained in Example 1-(XX), 64 mg of Boc-Cys(MBzℓ)-Phe-Gly-OH, 42 mg of HONB and 161 mg DCC, the same procedure as in Example 1-(XIX) was conducted to obtain the end product. Yield 302 mg (88.5%) m.p. 205 to 210°C (decomp.)

Analysis Calc'd for $C_{172}H_{228}N_{30}$ $O_{37}$ $S_6$: C,59.01; H,6.57; N,12.01; S,5.50
Found : C,59.33; H,6.83; N,12.09; S,5.30

(V) Production of H-Cys-Phe-Gly-Arg-Met-Asp-Arg-Ile-Gly-Ala-Gln-Ser-Gly-Cys-Asn-Ser-Phe-Arg-Tyr-OH ([des Gly$^{9,22}$ ,des Leu$^{21}$]-α-hANP[7-28])

Using 200 mg of the compound obtained in Example 5-(IV), HF treatment, air-oxidation and purification were conducted in the same manner of Example 1-(XXII) to obtain the end product. Yield 33.6 mg (26%)
Amino acid analysis : Asp 2.04, Ser 1.86, Glu 1.05, Gly 3.11, Ala 1.01, Half Cys 1.87, Met 1.00, Ile 1.00, Tyr 0.92, Phe 1.98, Arg 3.02

Example 6 Production of [des Gly9,20,22 ,des Ser $^{19}$ ,des Leu$^{21}$]-α-hANP[7-28]

(I) Production of Boc-Cys(MBzℓ)-Phe-Gly-Arg(Pme)-Met-Asp (OBzℓ)-Arg(Pme)-Ile-Gly-Ala-Glu-Cys(MBzℓ)-Asn-Ser(Bzℓ)-Phe-Arg(Pme)-Tyr(Bzℓ)-OBzℓ
Using 300 mg of the compound obtained in Example 3-(III), 61 mg of Boc-Cys(MBzℓ)-Phe-Gly-OH, 40 mg of HONB and 139 mg of DCC, the same procedure as in Example 1-(XIX) was conducted to obtain the end product. Yield 29.8 mg (87.0%) m.p. 212 to 217°C (decomp.)
    Analysis Calc'd for $C_{167}H_{220}N_{28}$ $O_{34}$ $S_6$: C,59.76; H,6.61; N,11.69; S,5.73
    Found : C,59.92; H,6.93; N,11.54; H,5.56

(II) Production of H-Cys-Phe-Gly-Arg-Met-Asp-Arg-Ile-Gly-Ala-Gln-Cys-Asn-Ser-Phe-Arg-Tyr-OH ([des Gly$^{9,20,22}$ ,des Ser$^{19}$,des Leu$^{21}$]-α-hANP[7-28])

Using 200 mg of the compound obtained in Example 6-(I), HF treatment, air-oxidation and purification were conducted in the same manner of Example 1-(XXII) to obtain the end product. Yield 26 mg (20%)
Amino acid analysis : Asp 2.03, Ser 0.88, Glu 1.04, Gly 2.06, Ala 1.02, Half Cys 1.88, Met 0.99, Ile 1.00, Tyr 0.93, Phe 1.94, Arg 3.10

Example 7 Production of [des Phe8,des Leu$^{21}$,des Gly$^{22}$]-α-hANP[7-28]

(I) Produciton of Boc-Cys(MBzℓ)-Gly-Gly-OH
In MeOH, 2.0 g of Boc-Gly-Gly-OBzℓ was subjected to $H_2$/Pd catalytic reduction. The resultant was subjected to condensation with 2.3 g of Boc-Cys(MBzℓ)-OH, 1.3 g of HONB and 1.5 g of DCC in the same manner of Example 1-(XVIII) to obtain the end product. Yield 1.47 g (34.5%) m.p. 125 to 130°C Rf$^2$ 0.43 [α]$_D^{25}$ -2.2° (c=0.93, in MeOH)
    Analysis Calc'd for $C_{20}$ $H_{29}$ $N_3O_7S$ : C,52.73; H,6.42; N, 9.22; S,7.04
    Found : C,52.87; H,6.64; N, 9.24; S,7.07

(II) Production of Boc-Cys(MBzℓ)-Gly-Gly-Arg(Pme)-Met-Asp (OBzℓ)-Arg(Pme)-Ile-Gly-Ala-Glu-Ser-Gly-Cys(MBzℓ)-Asn-Ser(Bzℓ)-Phe-Arg(Pme)-Tyr(Bzℓ)-OBzℓ
Using 300 mg of the compound obtained in Example 1-(XX), 75 mg of Boc-Cys(MBzℓ)-Gly-Gly-OH, 42 mg of HONB and 161 mg of DCC, the same procedure as in Example 1-(XIX) was conducted to obtain the end product. Yield 299 mg (89.9%) m.p. 198 to 210°C (decomp.)
    Analysis Calc'd for $C_{165}H_{222}N_{30}$ $O_{37}$ $S_6$ : C,58.11; H,6.56; N,12.32; S,5.64
    Found : C,58.35; H,6.78; N,12.03; S,5.41

(III) Production of H-Cys-Gly-Gly-Arg-Met-Asp-Arg-Ile-Gly-Ala-Gln-Ser-Gly-Cys-Asn-Ser-Phe-Arg-Tyr-OH ([des Phe$^8$, des Leu$^{21}$,des Gly$^{22}$]-α-hANP[7-28])

Using 200 mg of the compound obtained in Example 7-(II), HF treatment, air-oxidation and purification were conducted in the same manner of Example 1-(XXII) to obtain the end product Yield 28 mg (22%)
Amino acid analysis : Asp 2.02, Ser 1.88, Glu 1.04, Gly 4.06, Ala 1.01, Half Cys 1.88, Met 0.99, Ile 1.00, Tyr 0.91, Phe 1.01, Arg 3.03

Example 8 Production of [des Glys9,10,22 ,des Leu21]-α-hANP[7-28]

(I) Production of Boc-Phe-Arg(Pme)-Met-Asp(OBzℓ)-Arg(Pme)-Ile-Gly-OH

With 5 mℓ of TFA was treated 500 mg of Boc-Arg(Pme)-Met-Asp(OBzℓ)-Arg(Pme)-Ile-Gly-OH. The resulting amine component was dissolved in 5 mℓ of DMF, and the solution was cooled with ice, followed by addition of 0.13 mℓ of TEA. To the mixture was added 175 mg of Boc-Phe-ONB, which was stirred overnight. To the resultant was added 0.5 mℓ of AcOH, and the mixture was concentrated. To the concentrate was added AcOEt, and the resulting precipitates were collected by filtration. Yield 444 mg (80.1%) m.p. 172 to 178°C Rf$^2$ 0.44 [α] $^{25}_{D}$ -10.0° (c=1.0, in DMF)

Analysis Calc'd for $C_{72} H_{105} N_{13} O_{16} S_3$ : C, 57.47; H,7.03; N, 12.10; S,6.39
Found : C, 57.14; H,7.23; N, 12.12; S,6.47

(II) Production of Boc-Cys(MBzℓ)-Phe-Arg(Pme)-Met-Asp(OBzℓ)-Arg(Pme)-Ile-Gly-OH

Using 350 mg of Boc-Phe-Arg(Pme)-Met-Asp(OBzℓ)-Arg(Pme)-Ile-Gly-OH and Boc-Cys(MBzℓ)-ONB (prepared from 87 mg of Boc-Cys(MBzℓ)-OH, 48 mg of HONB and 55mg of DCC), the same procedure as in Example 8-(I) was conducted to obtain the end product, which was precipitated with hydrous acetonitrile and collected by filtration as precipitates. Yield 301 mg (74.9%) m.p. 189 to 196°C (decomp.) Rf$^2$ 0.47 [α] $^{25}_{D}$ -12.6° (c=1.0, in DMF)

Analysis Calc'd for $C_{83} H_{118} N_{14} O_{18} S_4 \bullet 3/2H_2O$ :C,56.80; H,6.95; N,11.17; S,7.31
Found :C,56.84; H,6.74; N,11.46; S,7.16

(III) Production of Boc-Cys(MBzℓ)-Phe-Arg(Pme)-Met-Asp(OBzℓ)-Arg(Pme)-Ile-Gly-Ala-Glu-Ser-Gly-Cys(MBzℓ)-Asn-Ser(Bzℓ)- Phe-Arg(Pme)-Tyr(Bzℓ)-OBzℓ

Using 300 mg of the compound obtained in Example 1-(XIX), 310 mg of Boc-Cys(MBzℓ)-Phe-Arg(Pme)-Met-Asp(OBzℓ)-Arg(Pme)-Ile-Gly-OH, 65 mg of HONB and 222 mg of DCC, the same procedure as in Example 1-(XIX) was conducted to obtain the end product. Yield 532 mg (94.7%) m.p. 201 to 210°C (decomp.)

Analysis Calc'd for $C_{170}H_{225}N_{29} O_{36} S_6$: C,59.30; H,6.59; N,11.80; S,5.59
Found : C,59.47; H,6.72; N,11.59; S,5.41

(IV) Production of H-Cys-Phe-Arg-Met-Asp-Arg-Ile-Gly-Ala-Gln-Ser-Gly-Cys-Asn-Ser-Phe-Arg-Tyr-OH ([des Gly$^{9,10,22}$, des Leu$^{21}$]-α-hANP[7-28])

Using 200 mg of the compound obtained in Example 8-(III), HF treatment, air-oxidation and purification were conducted in the same manner of Example 1-(XXII) to obtain the end product Yield 25 mg (19%)
Amino acid analysis : Asp 2.05, Ser 1.87, Glu 1.06, Gly 2.08, Ala 1.02 Half Cys 1.88, Met 0.98, Ile 1.00, Tyr 0.91, Phe 1.93, Arg 3.03

Example 9 Production of [des Phe,des Gly9,10,22,des Leu21]-α-hANP[7-28]

(I) Production of Boc-Cys(MBzℓ)-Gly-Arg(Pme)-Met-Asp(OBzℓ)-Arg(Pme)-Ile-Gly-Ala-Gln-Ser-Gly-Cys(MBzℓ)-Asn-Ser(Bzℓ)-Phe-Arg(Pme)-Tyr(Bzℓ)-OBzℓ

Using 300 mg of the compound obtained in Example 1-(XX), Boc-Cys(MBzℓ)-Gly-OH [prepared from 85 mg of Boc-Cys(MBzℓ)-Gly-OH•DCHA], 42 mg of HONB and 161 mg of DCC, the same procedure as in Example 1-(XIX) was conducted to obtain the end prodcut. Yield 258 mg (78.7%) m.p. 199 to 210°C (decomp.)

Analysis Calc'd for $C_{163}H_{219}N_{29} O_{36} S_6$ : C,58.38; H,6.58; N,12.12; S.5.74
Found : C,58.52; H,6.77; N,11.93; S,5.51

(II) Production of H-Cys-Arg-Met-Asp-Arg-Ile-Gly-Ala-Gln-Ser-Gly-Cys-Asn-Ser-Phe-Arg-Tyr-OH ([des Phe$^8$,des Gly$^{9\text{–}22}$ ,des Leu$^{21}$]-α-hANP[7-28]

Using 200 mg of the compound obtained in Example 9-(I), HF treatment, air-oxidation and purification were conducted in the same manner of Example 1-(XXII) to obtain the end product. Yield 26 mg (20%) Amino acid analysis : Asp 2.03, Ser 1.88, Glu 1.06, Gly 3.07, Ala 1.01, Half Cys 1.89, Met 1.00, Ile 1.00, Tyr 0.90, Phe 1.01, Arg 3.02

Example 10 Prodcution of [Cys$^9$,des Ser$^{19}$ ,des Gly$^{20,22}$,des Leu$^{21}$]-$\alpha$-hANP[8-28]

(I) Production of Boc-Phe-Cys(MBz$\ell$)-Gly-Arg(Pme)-Met-Asp (OBz$\ell$)-Arg(Pme)-Ile-Gly-Ala-Gln-Cys(MBz$\ell$)-Asn-Ser(Bz$\ell$)-Phe-Arg(Pme)-Tyr(Bz$\ell$)-OBz$\ell$

Using 300 mg of the compound obtained in Example 3-(III), 6.1 mg of Boc-Phe-Cys(MBz$\ell$)-Gly-OH, 40 mg of HONB and 139 mg of DCC, the same procedure as in Example 1-(XIX) was conducted to obtain the end product. Yield 249 mg (72.6%) m.p. 208 to 216°C (decomp.)

Analysis Calc'd for $C_{167}H_{220}N_{28}O_{34}S_6$ : C,59.76; H,6.61; N,11.69; S,5.73 Found : C,60.01; H,6.87; N 11.43; S,5.46

(II)  Production of H-Phe-Cys-Gly-Arg-Met-Asp-Arg-Ile-Gly-Ala-Gln-Cys-Asn-Ser-Phe-Arg-Tyr-OH ([Cys$^9$,des Ser$^{19}$,des Gly$^{20,22}$ ,des Leu$^{21}$]-$\alpha$-hANP[8-28])

Using 200 mg of the compound obtained in Example 10-(I), HF treatment, air-oxidation and purification were conducted in the same manner of Example 1-(XXII) to obtain the end product. Yield 24 mg (19%) Amino acid analysis : Asp 1.04, Ser 0.89, Glu 1.03, Gly 2.04 Ala 1.01, Half Cys 1.87, Met 0.99, Ile 1.00, Tyr 0.92, Phe 1.93, Arg 3.03

Example 11 Production of [des Gly$^{9,20,22}$,des Leu$^{21}$]-$\alpha$-hANP [7-28]

(I) Production of Boc-Cys(MBz$\ell$)-Phe-Gly-Arg(Pme)-Met-Asp (OBz$\ell$)-Arg(Pme)-Ile-Gly-Ala-Gln-Ser(Bz$\ell$)-Cys(MBz$\ell$)-Asn-Ser(Bz$\ell$)-Phe-Arg(Pme)-Tyr(Bz$\ell$)-OBz$\ell$

Using 300 mg of the compound obtained in Example 2-(IV), 61 mg of Boc-Cys(MBz$\ell$)-Phe-Gly-OH, 42 mg of HONB and 192 mg of DCC, the same procedure as in Example 1-(XIX) was conducted to obtain the end product. Yield 327 mg (93.4%) m.p. 215 to 222°C (decomp.)

Analysis Calc'd for $C_{177}H_{231}N_{29}O_{42}S_6$: C,58.57; H,6.42; N,11.19; S,5.30 Found : C,58.76; H,6.68; N11.03; S,5.07

(II)  Production of H-Cys-Phe-Gly-Arg-Met-Asp-Arg-Ile-Gly-Ala-Gln-Ser-Cys-Asn-Ser-Phe-Arg-Tyr-OH ([des Gly$^{9,20,22}$ , des Leu$^{21}$]-$\alpha$-hANP[7-28])

Using 200 mg of the compound obtained in Example 11-(I), HF treatment, air-oxidation and purification were conducted in the same manner of Example 1-(XXII) to obtain the end product. Yield 28 mg (22%) Amino acid analysis : Asp 2.04, Ser 1.87, Glu 1.06, Gly 2.10, Ala 1.01, Half Cys 1.78, Met 1.00, Ile 1.00, Tyr 0.93, Phe 1.96, Arg 3.03

Example 12 Production of [Cys$^9$,des Gly$^{20,22}$,des Leu$^{21}$]-$\alpha$-hANP [8-28]

(I) Production of Boc-Phe-Cys(MBz$\ell$)-Gly-Arg(Pme)-Met-Asp (OBz$\ell$)-Arg(Pme)-Ile-Gly-Ala-Gln-Ser(Bz$\ell$)-Cys(MBz$\ell$)-Asn-Ser(Bz$\ell$)-Phe-Arg(Pme)-Tyr(Bz$\ell$)-OBz$\ell$

Using 300 mg of the compound obtained in Example 2-(IV), 61 mg of Boc-Phe-Cys(MBz$\ell$)-Gly-OH, 42 mg of HONB and 192 mg of DCC, the same procedure as in Example 1-(XIX) was conducted to obtain the end product. Yield 314 mg (89.6%) m.p. 215 to 222°C (decomp.)

Analysis Calc'd for $C_{177}H_{231}N_{29}O_{42}S_6$: C,58.57; H,6.42; N,11.19; S,5.30 Found : C,58.83; H,6.71; N,10.95; S,5.01

(II)  Production of H-Phe-Cys-Gly-Arg-Met-Asp-Arg-Ile-Gly-Ala-Gln-Ser-Cys-Asn-Ser-Phe-Arg-Tyr-OH ([Cys$^9$,des Gly$^{20,22}$ ,des Leu$^{21}$]-α-hANP[8-28]

Using 200 mg of the compound obtained in Example 12-(I), HF treatment, air-oxidation and purification were conducted in the same manner of Example 1-(XXII). Yield 26 mg (21%)
Amino acid analysis : Asp 2.03, Ser 1.88, Glu 1.04, Gly 2.07, Ala 1.00, Half Cys 1.87, Met 0.99, Ile 1.00, Tyr 0.92, Phe 1.97, Arg 3.02

Example 13 Production of 3-phenylpropionyl-[des Phe$^8$,des Gly$^{9,22}$,des Leu$^{21}$]-α-hANP[7-28]

(I) Production of 3-phenylpropionyl-Cys(MBzℓ)-Gly-OH•DCHA
Using 1.0 g of Boc-Cys(MBzℓ)-Gly-OH•DCHA and 622 mg of 3-phenylpropionyl-ONB, the same procedure as in Example 1 -(XVIII) was conducted to obtain the end product, to which was added 0.34 mℓ of DCHA, followed by crystallization from ether. The crystals were collected by filtration. Yield 806 mg(76.6%) m.p. 144 to 147°C Rf$^2$ 0.65 [α] $^{25}_D$  -31.6° (c=1.0, in MeOH)
Analysis Calc'd for C$_{34}$ H$_{49}$ N$_3$O$_6$S : C,65.04; H,7.87 N, 6.69; S,5.11
Found : C,65.45; H,7.88; N, 6.77; S,5.12

(II) Production of 3-phenylpropionyl-Cys(MBzℓ)-Gly-Arg(Pme)-Met-Asp(OBzℓ)-Arg(Pme)-Ile-Gly-Ala-Gln-Ser-Gly-Cys(MBzℓ)-Asn-Ser(Bzℓ)-Phe-Arg(Pme)-Tyr(Bzℓ)-OBzℓ
Using 300 mg of the compound obtained in Example 1-(XX), 3-phenylpropionyl-Cys(MBzℓ)-Gly-OH (prepared from 72 mg of the corresponding DCHA salt), 39 mg of HONB and 178 mg of DCC, the same procedure as in Example 1-(XXI) was conducted to obtain the end product. Yield 266 mg(80.0%) m.p. 210 to 220°C (decomp.)
Analysis Calc'd for C$_{167}$H$_{219}$N$_{31}$ O$_{34}$ S$_6$ : C,59.03; H,6.51; N,12.78; S,5.66
Found : C,60.34; H,6.73; N,12.51; S,5.38

(III) Production of 3-phenylpropionyl-Cys-Gly-Arg-Met-Asp-Arg-Ile-Gly-Ala-Gln-Ser-Gly-Cys-Asn-Ser-Phe-Arg-Tyr-OH(3-phenylpropionyl-[des Phe$^8$,des Gly$^{9,22}$ ,des Leu$^{21}$]-α-hANP[7-28])

Using 200 mg of the compound obtained in Example 13-(II), HF treament, air-oxidation and purification were conducted in the same manner of Example 1-(XXII) to obtain the end product. Yield 24 mg (20%)
Amino acid analysis : Asp 2.03, Ser 1.90, Glu 1.02, Gly 3.03, Ala 1.00, Half Cys 1.88, Met 0.98, Ile 1.00, Tyr 0.92, Phe 0.98, Arg 3.02

Example 14 Production of [Aib$^6$,des Leu$^{21}$,des Gly$^{22}$]-α-hANP [6-28]

(I) Production of Boc-Aib-Cys(MBzℓ)-Phe-Gly-Gly-OH
Using 300 mg of Boc-Cys(MBzℓ)-Phe-Gly-Gly-OH, 112 mg of Boc-Aib-OH, 110 mg of HONB and 124 mg of DCC, the same procedure as in Example 1-(XVIII) was conducted to obtain the end product. Yield 240 mg(69.8%) m.p. 84 to 86°C Rf$^2$ 0.34 [α]    -38.8° (c=1.0 in DMF)
Analysis Calc'd for C$_{33}$ H$_{45}$ N$_5$O$_9$S : C,57.63; H,6.59; N,10.18; S,4.66
Found : C,57.87; H,6.95; N, 9.81; S,4.76

(II) Production of Boc-Aib-Cys(MBzℓ)-Phe-Gly-Gly-Arg(Pme)-Met-Asp(OBzℓ)-Arg(Pme)-Ile-Gly-Ala-Gln-Ser-Gly-Cys(MBzℓ)-Asn-Ser(Bzℓ)-Phe-Arg(Pme)-Tyr(Bzℓ)-OBzℓ
Using 300 mg of the compound obtained in Example 1-(XX), 74 mg of Boc-Aib-Cys(MBzℓ)-Phe-Gly-Gly-OH, 38 mg of HONB and 178 mg of DCC, the same procedure as in Example 1-(XXI) was 30 conducted to obtain the end product. Yield 331 mg(93.0%) m.p. 205 to 220°C (decomp.)
Analysis Calc'd for C$_{178}$H$_{238}$N$_{32}$ O$_{38}$ S$_6$: C,58.95; H1,6.62; N,12.36; S,5.31
Found : C,59.26; H,6.93; N,12.01; S,5.06

(III) Production of H-Aib-Cys-Phe-Gly-Gly-Arg-Met-Asp-Arg-Ile-Gly-Ala-Gln-Ser-Gly-Cys-Asn-Ser-Phe-Arg-Tyr-OH([Aib$^6$, des Leu$^{21}$,des Gly$^{22}$]-α-hANP[6-28])

Using 200 mg of the compound obtained in Example 14-(II), HF treatment, air-oxidation and purification were conducted in the same manner of Example 1-(XXII) to obtain the end product. Yield 18 mg(14%)
Amino acid analysis : Asp 2.03, Ser 1.88, Glu 1.04, Gly 4.12, Ala 1.00, Half Cys 1.89, Met 0.98, Ile 1.00, Tyr 0.92, Phe 1.93, Arg 3.03, Aib 0.95

Example 15 Production of [Aib$^6$,des Ser$^{19}$,des Gly$^{20,22}$, des Leu$^{21}$-α-hANP[6-28]

(I) Production of
Boc-Aib-Cys(MBz$\ell$)-Phe-Gly-Gly-Arg(Pme)-Met-Asp(OBz$\ell$)-Arg(Pme)-Ile-Gly-Ala-Gln-Cys(MBz$\ell$)-Asn-Ser(Bz$\ell$)-Phe-Arg(Pme)-Tyr(Bz$\ell$)-OBz$\ell$
Using 300 mg of the compound obtained in Example 3-(III), 77 mg of Boc-Aib-Cys(MBz$\ell$)-Phe-Gly-Gly-OH, 40 mg of HONB and 185 mg of DCC, the same procedure as in Example 1-(XXI) was conducted to obtain the end product. Yield 262 mg (73.3%) m.p. 215 to 225°C(decomp.)
Analysis Calc'd for $C_{173}H_{230}N_{30}O_{35}S_6$ : C,59.67; H,6.66; N,12.07; S,5.52
Found : C,60.03; H,6.89; 11.84; S,5.28

(II) Production of H-Aib-Cys-Phe-Gly-Gly-Arg-Met-Asp-Arg-Ile-Gly-Ala-Gln-Cys-Asn-Ser-Phe-Arg-Tyr-OH([Aib$^6$,des Ser$^{19}$, des Gly$^{20,22}$,des Leu$^{21}$]-α-hANP[6-28])

Using 200 mg of the compound obtained in Example 15-(I), HF treatment, air-oxidation and purification were conducted in the same manner of Example 1-(XXII) to obtain the end product. Yield 16 mg(12%)
Amino acid analysis : Asp 2.06, Ser 0.91, Glu 1.03, Gly 1.07, Ala 1.00, Half Cys 1.88, Met 0.99, Ile 1.00, Tyr 0.92, Phe 1.96, Arg 3.02, Aib 0.96

Example 16 Production of [Gly$^6$, des Leu$^{21}$, des Gly$^{22}$]-αhANP [6-28]

(I) Production of Boc-Gly-Cys(MBz$\ell$)-Phe-Gly-Gly-OH
Using 300 mg of Boc-Cys(MBz$\ell$)-Phe-Gly-Gly-OH, 96 mg of Boc-Gly-OH, 110 mg of HONB and 120 mg of DCC, the same procedure as in Example 1-(XVIII) was conducted to obtain the end product.
Yield 266 mg (81.1%)
m.p. 95 to 100°C    Rf$^2$ 0.31
[α] $_D^{25}$ -20.8° (c = 1.0, in DMF)
Analysis Calc'd for $C_{31}H_{41}N_5O_9S$: C,56.44; H,6.24; N,10.62; S, 4.86
Found : C,56.54; H,6.52;N,10.49; S, 4.67

(II) Production of
Boc-Gly-Cys(MBz$\ell$)-Phe-Gly-Gly-Arg(Pme)-Met-Asp(OBz$\ell$)-Arg(Pme)-Ile-Gly-Ala-Gln-Ser-Gly-Cys(MBz$\ell$)-Asn-Ser(Bz$\ell$-Phe-Arg(Pme)-Tyr(Bz$\ell$)-OBz$\ell$
Using 300 mg of the compound obtained in Example 1-(XX), 71 mg of Boc-Gly-Cys(MBz$\ell$)-Phe-Gly-Gly-OH, 38 mg of HONB and 178 mg of DCC, the same procedure as in Example 1-(XXI) was conducted to obtain the end product.
Yield 312 mg (89.2%)
m.p. 213-222°C (decomp.)
Analysis Calc'd for $C_{176}H_{234}N_{32}O_{39}S_6$: C,58.49; H,6.53; N,12.40; S, 5.32
Found C,58.93; H,6.97; N,12.15; S, 5.14

(III)   Production of H–Gly–Cys–Phe–Gly–Gly–Arg–Met–Asp–

Arg–Ile–Gly–Ala–Gln–Ser–Gly–Cys–Asn–Ser–Phe– Arg–Tyr–

OH ([Gly$^6$, des Leu$^{21}$, des Gly$^{22}$] –αhANP[6-28])

Using 200 mg of the compound obtained in Example 16-(II), HF treatment, air -oxidation and purification were conducted in the same manner of Example 1-(XXII) to obtain the end product.
Yield 36 mg (26.9%)
Amino acid analysis: Asp 2.01, Ser 1.88, Glu 1.01, Gly 4.87, Ala 0.98, Half Cys 1.91, Met 1.03, Ile 1.00, Tyr 0.94, Phe 2,03, Arg 3.08

Example 17 Production of [Ala$^6$, des Leu$^{21}$, des Gly $^{22}$]-αhANP [6-28]

(I)Production of Boc-Ala-Cys(MBz$\ell$)-Phe-Gly-Gly-OH
Using 300 mg of Boc-Cys(MBz$\ell$)-Phe-Gly-Gly-OH, 104 mg of Boc-Ala-OH, 110 mg of HONB and 120 mg of DCC, the same procedure as in Example 1-(XVIII) was conducted to obtain the end product.
Yield 265 mg (79.0%)
m.p. 133 - 137°C    Rf$^2$ 0.34
[α] $^{25}_D$  - 27.0° (c =1.0, in DMF)
    Analysis Calc'd for C$_{32}$H$_{43}$N$_5$O$_9$S: C,57.04; H,6.43; N,10.39; S, 4.76
    Found: C, 57.48; H, 6.81; N, 10.16; S, 4.12=

(II) Production of
Boc-Ala-Cys(MBz$\ell$)-Phe-Gly-Gly-Arg(Pme)-Met-Asp(OBz$\ell$)-Arg(P:ëGly-Ala-Gln-Ser-Gly-Cys(MBz$\ell$)-Asn-Ser(Bz$\ell$)-Phe-Arg(Pme)-Tyr(Bz$\ell$)-OBz$\ell$
Using 300 mg of the compound obtained in Example 1-(XX), 72 mg of Boc-Ala-Cys(MBz$\ell$)-Phe-Gly-Gly-OH, 38 mg of HONB and 178 mg of DCC, the same procedure as in Example 1-(XXI) was conducted to obtain the end product.
Yield 320 mg (90.3%)
m.p. 216 - 225°C (decomp.)
    Analysis Calc'd for C$_{177}$H$_{236}$N$_{32}$O$_{39}$S$_6$: C,58.59; H,6.56; N,12.35; S, 5.30
    Found C,59.17; H,7.03; N,12.18; S, 5.20

(III)   Production of H–Ala–Cys–Phe–Gly–Gly–Arg–Met–Asp–

Arg–Ile–Gly–Ala–Gln–Ser–Gly–Cys–Asn–Ser–Phe–Arg–
Leu21,

Tyr–OH ([Ala$^6$,des Leu$^{21}$, des Gly$^6$]–αhANP[6-28])

Using 200 mg of the compound obtained in Example 17-(II), HF treatment, air -oxidation and purification were conducted in the same manner of Example 1-(XXII) to obtain the end product.
Yield 31 mg (22.9%)
Amino acid analysis: Asp 1.98, Ser 1.86, Glu 1.04, Gly 4.05, Ala 2.01, Half Cys 1.92, Met 1.03, Ile 1.00, Tyr 0.92, Phe 2.05, Arg 3.06,

Example 18 Production of [Val$^6$, des Leu$^{21}$, des Gly $^{22}$]-αhANP [6-28]

(I) Production of Boc-Val-Cys(MBz$\ell$)-Phe-Gly-Gly-OH
Using 300 mg of Boc-Cys(MBz$\ell$)-Phe-Gly-Gly-OH, 119mg of Boc-Val-OH, 110 mg of HONB and 120 mg of DCC, the same procedure as in Example 1-(XVIII) was conducted to obtain the end product.
Yield 324 mg (92.7%)
m.p. 146 - 150°C    Rf$^2$ 0.42
[α] $^{25}_D$ - 20.7° (c =1.0, in DMF)
    Analysis Calc'd for C$_{34}$H$_{47}$N$_5$O$_9$S: C,58.19; H,6.75; N,9.98; S, 4.57
    Found : C,58.43; H,6.94; N,10.08; S, 4.26

(II) Production of
Boc-Val-Cys(MBz$\ell$)-Phe-Gly-Gly-Arg(Pme)-Met-Asp(OBz$\ell$)-Arg(Pme)-Ile-Gly-Ala-Gln-Ser-Gly-Cys(MBz$\ell$)-Asn-Ser(Bz$\ell$)-Phe-Arg(Pme)-Tyr(Bz$\ell$)-OBz$\ell$

Using 300 mg of the compound obtained in Example 1-(XX) , 75 mg of Boc-Val-Cys(MBz$\ell$)-Phe-Gly-Gly-OH, 38 mg of HONB and 178 mg of DCC, the same procedure as in Example 1-(XXI) was conducted to obtain the end product.
Yield 319 mg (89.3%)
m.p. 220 - 230°C (decomp.)
Analysis Calc'd for $C_{179}H_{24}ON_{32}O_{39}S_6$: C,58.80; H,6.62; N,12.26; S, 5.26
Found C,59.03; H,6.96; N,12.03; S, 5.03

(III)    Production of H-Val-Cys-Phe-Gly-Gly-Arg-Met-Asp-

Arg-Ile-Gly-Ala-Gln-Ser-Gly-Cys-Asn-Ser-Phe-Arg-des

Tyr-OH ([Val$^6$, Leu$^{21}$, des Gly$^6$]-$\alpha$hANP[6-28])

Using 200 mg of the compound obtained in Example 18-(II), HF treatment, air-oxidation and purification were conducted in the same manner of Example 1-(XXII) to obtain the end product.
Yield 22 mg (16.8%)
Amino acid analysis: Asp 2.02, Ser 1.90, Glu 0.97, Gly 3.96, Ala 1.02, Half Cys 1.93, Met 1.02, Ile 1.00, Tyr 0.95, Phe 1.99, Arg 3.04, Val 0.97

Example 19 Production of [Ile$^6$, des Leu$^{21}$, des Gly $^{22}$]-$\alpha$hANP [6-28]

(I) Production of Boc-Ile-Cys(MBz$\ell$)-Phe-Gly-Gly-OH
Using 300 mg of Boc-Cys(MBz$\ell$)-Phe-Gly-Gly-OH, 131 mg of Boc-Ile-OH•1/2 H2O, 110 mg of HONB and 120 mg of DCC, the same procedure as in Example 1-(XVIII) was conducted to obtain the end product.
Yield 296 mg (83.0%)
m.p. 146 - 150°C    Rf$^2$ 0.45
[$\alpha$] $^{25}_D$ - 22.3° (c = 1.0, in DMF)
Analysis Calc'd for $C_{35}H_{49}N_5O_9S$: C,58.72; H,6.90; N,9.78; S, 4.48
Found : C,58.53; H,6.94; N,9.65; S, 4.45

(II) Production of
Boc-Ile-Cys(MBz$\ell$)-Phe-Gly-Gly-Arg(Pme)-Met-Asp(OBz$\ell$)-Arg(Pme)-Ile-Gly-Ala-Gln-Ser-Gly-Cys(MBz$\ell$)-Asn-Ser(Bz$\ell$)-Phe-Arg(Pme)-Tyr(Bz$\ell$)-OBz$\ell$

Using 300 mg of the compound obtained in Example 1-(XX), 77 mg of Boc-Ile-Cys(MBz$\ell$)-Phe-Gly-Gly-OH, 38 mg of HONB and 178 mg of DCC, the same procedure as in Example 1-(XXI) was conducted to obtain the end product.
Yield 323 mg (90.1%)
m.p. 218 - 229°C (decomp.)
Analysis Calc'd for $C_{180}H_{242}N_{32}O_{39}S_6$: C,58.90; H,6.65; N,12.21; S, 5.24
Found C,60.07; H,6.81; N,12.03; S, 5.12

(III)    Production of H-Ile-Cys-Phe-Gly-Gly-Arg-Met-Asp-

Arg-Ile-Gly-Ala-Gln-Ser-Gly-Cys-Asn-Ser-Phe-Arg-

Tyr-OH ([Ile$^6$,des Leu$^{21}$, des Gly$^{22}$] -$\alpha$hANP[6-28])

Using 200 mg of the compound obtained in Example 19-(II), HF treatment, air -oxidation and purification were conducted in the same manner of Example 1-(XXII) to obtain the end product.
Yield 33 mg (24.8%)
Amino acid analysis: Asp 2.02, Ser 1.87, Glu 1.01, Gly 4.05, Ala 1.02, Half Cys 1.93, Met 0.97, Ile 2.00, Tyr 0.91, Phe 1.94, Arg 3.07

Example 20 Production of Ac-[des Leu21, des Gly 22]-αhANP [7-28]

(I) Production of Ac-Cys(MBzℓ)-Phe-Gly-Gly-OH
Using 300 mg of Boc-Cys(MBzℓ)-Phe-Gly-Gly-OH and 122 mg of Ac-ONB, the same procedure as in Example 1-(XVIII) was conducted to obtain the end product.
Yield 144 mg (52.9%)
m.p. 206 - 207°C (decomp.)    Rf2 0.22
[α] $_D^{25}$ -35.8° (c=0.9, in DMF)
Analysis Calc'd for C26H32N4O7S: C,57.34; H,5.92; N,10.29; S, 5.89
Found : C,57.60; H,5.93; N,9.96; S, 6.29

(II) Production of
Ac-Cys(MBzℓ)-Phe-Gly-Gly-Arg(Pme)-Met-Asp(OBzℓ)-Arg(Pme)-Ile-Gly-Ala-Gln-Ser-Gly-Cys(MBzℓ)-Asn-Ser(Bzℓ)-Phe-Arg(Pme)-Tyr(Bzℓ)-OBzℓ
Using 300 mg of the compound obtained in Example 1-(XX), 58 mg of Ac-Cys(MBzℓ)-Phe-Gly-Gly-OH, 38 mg of HONB and 178 mg of DCC, the same procedure as in Example 1-(XXI) was conducted to obtain the end product.
Yield 286 mg (83.7%)
m.p. 217 - 227°C (decomp.)
Analysis Calc'd for $C_{171}H_{225}N_{31}O_{37}S_6$: C,58.69; H,6.48; N,12.41; S, 5.50
Found : C,59.16; H,6.58; N,12.24; S, 5.04

(III)   Production of Ac-Cys-Phe-Gly-Gly-Arg-Met-Asp-Arg-

Ile-Gly-Ala-Gln-Ser-Gly-Cys-Asn-Ser-Phe-Arg-Tyr-

OH (Ac-[des Leu21, des Gly22] -αhANP[7-28])

Using 200 mg of the compound obtained in Example 20-(II), HF treatment, air -oxidation and purification were conducted in the same manner of Example 1-(XXII) to obtain the end product.
Yield 27 mg (19.8%)
Amino acid analysis: Asp 2.03, Ser 1.88 Glu 0.99, Gly 4.05, Ala 1.02, Half Cys 1.85, Met 1.03, Ile 1.00, Tyr 0.90, Phe 2.02, Arg 2.98

Example 21 Production of n-Capryl-[des Leu21, des Gly 22]-αhANP [7-28]

(I) Production of n-Capryl-Cys(MBzℓ)-Phe-Gly-Gly-OH
Using 300 mg of Boc-Cys(MBzℓ)-Phe-Gly-Gly-OH and 200 mg of n-Capryl-ONB, the same procedure as in Example 1-(XVIII) was conducted to obtain the end product.
Yield 290 mg (88.3%)
m.p. 174 - 176°C,    Rf2 0.39
[α] $_D^{25}$ -29.8° (c=1.0, in DMF)
Analysis Calc'd for C34H48N4O7S.1/2 H2O:C,61.33; H,7.42; N,8.42; S, 4.82
Found : C,61.53; H,7.51; N,8.61; S, 5.00

(II) Production of
n-Capryl-Cys(MBzℓ)-Phe-Gly-Gly-Arg(Pme)-Met-Asp(OBZℓ)-Arg(Pme)-Ile-Gly-Ala-Gln-Ser-Gly-Cys(MBzℓ)-Asn-Ser(Bzℓ)-Phe-Arg(Pme)-Tyr(Bzℓ)-OBzℓ
Using 300 mg of the compound obtained in Example 1-(XX), 71 mg of n-Capryl-Cys(MBzℓ)-Phe-Gly-Gly-OH, 38 mg of HONB and 178 mg of DCC, the same procedure as in Example 1-(XXI) was conducted to obtain the end product.
Yield 295 mg (83.6%)
m.p. 217 - 227°C (decomp.)
Analysis Calc'd for $C_{179}H_{241}N_{31}O_{37}S_6$: C,59.53; H,6.73; N,12.01; S, 5.33
Found : C,60.02; H,7.16; N,11.91; S, 5.15

(III)    Production of n-Capryl-Cys-Phe-Gly-Gly-Arg-Met-

Asp-Arg-Ile-Gly-Ala-Gln-Ser-Gly-Cys-Asn-Ser-Phe-Arg-

Tyr-OH (n-Capryl-[des Leu21, des Gly22]-

αhANP[7-28])

Using 200 mg of the compound obtained in Example 21-(II), HF treatment, air -oxidation and purification were conducted in the same manner of Example 1-(XXII) to obtain the end product.
Yield 28 mg (20.3%)
Amino acid analysis: Asp 1.96, Ser 1.93, Gln 1.03, Gly 4.04, Ala 1.02, Half Cys 1.87, Met 0.96, Ile 1.00, Tyr 0.92, Phe 2.02, Arg 3.03

Example 22 Production of [Ile12, des Leu21,des Gly22]-αhANP[7-28]

(I) Production of Boc-Ile-Asp(OBzl)-Arg(Pme)-Ile-Gly-Oh
Using 1.0g of Boc-Asp(OBzl)-Arg(Pme)-Ile-Gly-OH-CHA and Boc-Ile-ONB (prepared from 276 mg of Boc-Ile-OH.1/2 $H_2O$, 230 mg of HONB and 262 mg of DCC), the same procedure as in Example 1-(XIII) was conducted to obtain the end product.
Yield 0.83 g (82.0%)
m.p. 176 - 178°C,     $Rf^2$ 0.47
$[\alpha]_D^{25}$ - 16.0° (C = O.6, in DMF)
    Analysis Calc'd for $C_{47}H_{72}N_8O_{12}S.H_2O$: C,56.95; H,7.52; N,11.30; S, 3.42;
    Found : C,57.11; H,7.34; N,11.41; S, 3.07

(II) Production of Boc-Arg(Pme)-Ile-Asp(OBzℓ)-Arg(Pme)-Ile-Gly-OH
Using 0.39 g of Boc-Ile-Asp(OBzl)-Arg(Pme)-Ile-Gly-OH, Boc-Arg(Pme)-ONB (prepared from 0.21 g of Boc-Arg(Pme)-OH 90 mg of HONB and 100 mg of DCC), the same procedure as in Example 1-(XVI) was conducted to obtain the end product.
Yield 0.33 g (61.6%)
m.p. 182 - 184°C $RF_2$ 0.51
$[\alpha]_D^{25}$ - 9.8° (C = 1.0, in DMF)
    Analysis Calc'd for $C_{64}H_{98}N_{12}O_{15}S_2$: C,57.38; H,7.37; N,12.55; S, 4.79
    Found : C,57.25; H,7.62; N,12.17; S,4.86.

(III) Production of
Boc-Arg(Pme)-Ile-Asp(OBzℓ)-Arg(Pme)-Ile-Gly-Ala-Gln-Ser-Gly-Cys(MBzl)-Asn-Ser(Bzl)-Phe-Arg(Pme)-Tyr(Bzl)-OBzl
Using 500 mg of the compound obtained in Example 1-(XIX), 474 mg of Boc-Arg(Pme)-Ile-Asp(OBzl)-Arg(Pme)-Ile-Gly-OH, 98 mg of HONB and 337 mg of DCC, the same procedure as in Example 1-(XIX) was conducted to obtain the end product.
Yield 793 mg (95.6%)
m.p. 217 - 222°C (decomp.), $Rf^2$ 0.51
$[\alpha]_D^{25}$ -13.1° (C = 1.0, in DMF)
    Analysis Calc'd for $C_{151}H_{205}N_{27}O_{33}S_4$: C,59.37; H,6.76; N,12.38; S, 4.20
    Found C,60.01; H,6.89; N,12.25; S,4.44.

(IV) Production of
Boc-Cys(MBzl)-Phe-Gly-Gly-Arg(Pme)-Ile-Asp(OBzℓ)-Arg(Pme)-Ile-Gly-Ala-Gln-Ser-Gly-Cys(MBzl)-Asn-Ser(Bzl)-Phe-Arg(Pme)-Tyr(Bzl)-OBzl
Using 600 mg of the compound obtained in Example 22-(III), 130 mg of Boc-Cys(MBzl)-Phe-Gly-Gly-OH, 80 mg of HONB and 360 mg of DCC, the same procedure as in Example 1-(XIX) was conducted to obtain the end product.
Yield 542 mg (77.9%)
m.p. 226 - 228°C (decomp.)
    Analysis Calc'd for $C_{175}H_{233}N_{31}O_{38}S_5$: C,59.39; H,6.64; N,12.27; S, 4.53
    Found C,59.28; H,7.02; N,12.08; S,4.23.

(V)  Production of H-Cys-Phe-Gly-Gly-Arg-Ile-Asp-Arg-Ile-

Gly-Ala-Gln-Ser-Gly-Cys-Asn-Ser-Phe-Arg-Tyr-

OH(Ile12, des Leu21, [des Gly22] -αhANP[7-28])

Using 200 mg of the compound obtained in Example 22-(IV), HF treatment, air-oxidation and purification were conducted in the same manner of in Example 1-(XXII) to obtain the end product.
Yield 39.3 mg (29.7%)
Amino acid analysis: Asp 2.03, Ser 1.84, Glu 0.99, Gly 3.91, Ala 0.98, Half Cys 1.88, Ile 2.00, Tyr 0.95, Phe 1.94, Arg 3.06

Example 23

Production of [N$\ell$e12, des Leu21, des G$\ell$y22]-αhANP [7-28]

(I) Production of Boc-N$\ell$e-Asp(OBzP)-Arg(Pme)-I$\ell$e-G$\ell$y-OH
Using 1.0 g of Boc-Asp(OBz$\ell$)-Arg(Pme)-I$\ell$e-G$\ell$y-OH•CHA and Boc-N$\ell$e-ONB(prepared from 495 mg of Boc-N$\ell$e-OH, 230 mg of HONB and 262 mg of DCC, the same procedure as in Example 1-(XIII) was conducted to obtain the end product.
Yield 1.03 g (97.0%)
m.p. 162 - 167°C    Rf2 0.43
[α] $_D^{25}$   -13.5° (C=1.0, in DMF)
Analysis Calc'd for $C_{47}H_{72}N_8O_{12}S$: C, 58.01; H, 7.46; N, 11.51; S, 3.29
Found: C, 57.85; H, 7.60; N, 11.36; S, 3.09

(II) Production of Boc-Arg(Pme)-N$\ell$e-Asp(OBz$\ell$)-Arg(Pme)-I$\ell$e-G$\ell$y-OH
Using 0.39 g of Boc-N$\ell$e-Asp(OBz$\ell$)-Arg(Pme)-I$\ell$e-G$\ell$y-OH and Boc-Arg(Pme)-ONB(prepared from 0.23 g of Boc-Arg(Pme)-OH, 95 mg of HONB and 109 mg of DCC, the same procedure as in Example 1-(XVI) was conducted to obtain the end product.
Yield 0.47 g (87.5%)
m.p. 172 - 176°C    Rf2 0.49
[α] $_D^{25}$   - 10.6° (C=1.0, in DMF)
Analysis Calc'd for $C_{64}H_{98}N_{12}O_{15}S_2$: C, 57.42; H, 7.30; N, 12.56; S, 4,79
Found: C, 57.51; H, 7.47; N, 12.32; S, 4.97

(III) Production of
Boc-Arg(Pme)-N$\ell$e-Asp(OBz$\ell$)-Arg(Pme--I$\ell$eG$\ell$y-A$\ell$a-G$\ell$n-Ser-G$\ell$y-Cys(MBz$\ell$)-Asn-Ser(Bz$\ell$)-Phe-Arg(Pme)-Tyr(Bz$\ell$)-OBz$\ell$
Using 500 mg of the compound obtained in Example 1-(XIX), 420 mg of Boc-Arg(Pme)-N$\ell$e-Asp(OBz$\ell$)-Arg(Pme)-I$\ell$e-G$\ell$y-OH, 108 mg of HONB and 495 mg of DCC, the same procedure as in Example 1-(XIX) was conducted to obtain the end product.
Yield 810 mg (95.8%)
m.p. 199 - 203°C (decomp.)    Rf2 0.33
[α] $_D^{25}$   -11.4° (C=1.0, in DMF)
Analysis Calc'd for $C_{151}H_{205}N_{27}O_{33}S_4$: C, 59.37; H, 6.76; N, 6.89; S, 4.20
Found: C, 59.16; H, 6.99; N, 7.01; S, 4.07

(IV) Production of
Boc-Cys(MBz$\ell$)-Phe-G$\ell$y-G$\ell$y-Arg(Pme)-N$\ell$e-Asp(OBz$\ell$)-Arg(Pme)-I$\ell$e-G$\ell$y-A$\ell$a-G$\ell$n-Ser-G$\ell$y-Cys(MBz$\ell$)-Asn-Ser(Bz$\ell$)-Phe-Arg(Pme)-Tyr(Bz$\ell$)-OBz$\ell$
Using 600 mg of the compound obtained in Example 23-(III), 130 mg of Boc-Cys(MBz$\ell$)-Phe-G$\ell$y-G$\ell$y-OH, 80mg of HONB and 360 mg of DCC, the same procedure as in Example 1-(XIX) was conducted to obtain the end product.
Yield 261 mg (75.1%)
m.p. 215 220°C (decomp.)
Analysis Calc'd for $C_{175}H_{233}N_{31}O_{38}S_5$: C, 59.39; H, 6.64; N, 12.27; S, 4.53
Found: C, 59.03; H, 6.72; N, 12.47; S, 4.36

(V) Production of H-Cys-Phe-Gly-Gly-Arg-Nℓe-Asp-Arg-Iℓe-

Gly-Aℓa-Gℓn-Ser-Gly-Cys-Asn-Ser-Phe-Arg-Tyr-OH

Using 200mg of the compound obtained in Example 23-(IV), HF treatment, air-oxidation and purification were conducted in the same manner of in Example 1-(XXII) to obtain the end product.
Yield 34.8 mg (23.5%)

Amino acid analysis: Asp 2.01, Ser 1.89, Gℓu 0.97, Gℓy 4.01, Aℓa 0.99, Half Cys 1.86, Iℓe 1.00, Tyr 0.94, Phe 1.99, Arg 3.03, Nℓe 0.98

## Claims

1. A peptide of the formula

A-Cys-B-Arg-C-Asp-Arg-Ile-Gly-Ala-E-Cys-Asn-Ser-Phe-
Arg-Tyr-OH

wherein A is hydrogen or a hydrocarbon acyl having 2 to 10 carbon atoms which may be substituted with an amino group, B is Phe, Gly, Phe-Gly, Gly-Gly or Phe-Gly-Gly, C is a neutral $\alpha$-amino acid residue, and E is Gln, Gln-Ser or Gln-Ser-Gly, respectively, or a salt thereof.

2. The peptide as claimed in claim 1, wherein A is hydrogen.
3. The peptide as claimed in claim 1, wherein A is Phe.
4. The peptide as claimed in claim 1, wherein B is Phe-Gly-Gly.
5. The peptide as claimed in claim 1, wherein B is Gly.
6. The peptide as claimed in claim 1, wherein C is Met.
7. The peptide as claimed in claim 1, wherein E is Gln-Ser-Gly.
8. The peptide as claimed in claim 1, wherein E is Gln.
9. The peptide as claimed in claim 1, wherein A is hydrogen, B is Phe-Gly-Gly, C is Met and E is Gln-Ser-Gly.
10. A method for producing a peptide of the formula

A-Cys-B-Arg-C-Asp-Arg-Ile-Gly-Ala-E-Cys-Asn-Ser-Phe-
Arg-Tyr-OH

wherein A is hydrogen or a hydrocarbon acyl having 2 to 10 carbon atoms which may be substituted with an amino group, B is Phe, Gly, Phe-Gly, Gly-Gly or Phe-Gly-Gly, C is a neutral $\alpha$-amino acid residue, and E is Gln, Gln-Ser or Gln-Ser-Gly, respectively, or a salt thereof, which comprises subjecting a peptide of the formula:

A-Cys-B-Arg-C-Asp-Arg-Ile-Gly-Ala-E-Cys-Asn-Ser-Phe-Arg-Tyr-OH

wherein A, B, C and E are of the same meaning as defined above or a salt thereof, to oxidation.